(19) **Europäisches Patentamt / European Patent Office / Office européen des brevets**

(11) **EP 4 693 771 A1**

(12) **EUROPÄISCHE PATENTANMELDUNG**

(43) Veröffentlichungstag:
**11.02.2026 Patentblatt 2026/07**

(21) Anmeldenummer: **24192874.6**

(22) Anmeldetag: **05.08.2024**

(51) Internationale Patentklassifikation (IPC):
**H01S 5/34** *(2006.01)* **H01S 5/40** *(2006.01)*
**G01N 21/17** *(2006.01)* **G01N 21/25** *(2006.01)*

(52) Gemeinsame Patentklassifikation (CPC):
**G01N 21/1702; A61B 5/00; G01N 21/171;
G01N 21/1717; G01N 21/255; H01S 5/005;
H01S 5/3401; H01S 5/4012; H01S 5/405;
H01S 5/4087; H01S 5/423;** G01N 2021/1708;
G01N 2021/1725

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH MA MD TN**

(71) Anmelder: **DiaMonTech AG**
**10245 Berlin (DE)**

(72) Erfinder:
• **JANIK, Sergius**
**10245 Berlin (DE)**
• **KALUZA, Michael**
**10245 Berlin (DE)**
• **LUBINSKI, Thorsten**
**10245 Berlin (DE)**

(74) Vertreter: **Lucke, Andreas**
**Boehmert & Boehmert
Anwaltspartnerschaft mbB
Pettenkoferstrasse 22
80336 München (DE)**

(54) **ANREGUNGSQUELLE ZUR EINSTRAHLUNG VON ANREGUNGSSTRAHLUNG IN EIN VOLUMEN SOWIE VORRICHTUNG ZUM NACHWEIS EINES STOFFES MIT EINER SOLCHEN ANREGUNGSQUELLE**

(57) Beschrieben wird eine Anregungsquelle (26, 200) zur Einstrahlung von Anregungsstrahlung (18, 201) in ein Volumen, mit einer Mehrzahl von Anregungsquellenelementen (200a, 200b, 200c, 200d, 200e, 200f, 200g, 200h, 200i, 200j), von denen jedes eine Anregungsstrahlung (18, 201) in einem ihm zugeordneten Wellenlängenbereich in einer gemeinsamen Strahlrichtung (202) aussendet, wobei wenigstens eine erste und eine zweite Gruppe (203, 204) mit jeweils mehreren Anregungsquellenelementen vorgesehen sind, wobei die Anregungsquellenelemente jeder Gruppe auf einer gemeinsamen, quer zur Strahlrichtung, insbesondere senkrecht zur Strahlrichtung, angeordneten Fläche oder Linie angeordnet sind und wobei die verschiedenen Gruppen von Anregungsquellenelementen in Strahlrichtung voneinander beabstandet sind.

Fig. 14

Fig. 15

**Beschreibung**

Die vorliegende Erfindung liegt auf dem Gebiet der Lasertechnik, der Messtechnik und der Medizintechnik. Sie betrifft eine Anregungsquelle, die beispielsweise als Laserarray ausgebildet sein kann, sowie eine Vorrichtung zum Nachweis eines Stoffs oder zur Messung einer Stoffkonzentration. Die Anregungsquelle weist eine Mehrzahl von Anregungsquellenelementen auf, die in einem Ausführungsbeispiel als einzelne Quantenkaskadenlaser ausgebildet sein können.

Eines der durch diese Gegenstände ermöglichten Verfahren umfasst beispielsweise einen Messvorgang, bei dem die durch Absorption einer Anregungsstrahlung in einer Probe, allgemeiner in einem Volumen, entstehende Temperaturerhöhung und die erzeugten Wärme- und/oder Druckwellen nachgewiesen werden können. Anregungsstrahlung wird in das Volumen, beispielsweise ein menschliches oder tierisches Gewebe gestrahlt, um von dem darin enthaltenen Analyten absorbiert zu werden, wobei die Intensität der Anregungsstrahlung zeitmoduliert sein kann und wobei die Anregungsstrahlung Strahlung verschiedener Analyt-charakteristischer Wellenlängen umfassen kann.

Unter Analyt-charakteristischen Wellenlängen werden hier Wellenlängen verstanden, die die Bestimmung des Vorhandenseins eines Analyten durch wellenlängenselektive Absorption ermöglichen und somit die Grundlage der Analyse bilden.

Als solche können Analyt-charakteristische Wellenlängen insbesondere Wellenlängen umfassen, die Absorptionsmaxima des Analyten entsprechen. Weitere Analyt-charakteristische Wellenlängen können Wellenlängen sein, die lokalen Minima zwischen zwei Absorptionspeaks entsprechen. Genauer gesagt ist oft die Differenz zwischen einem lokalen Absorptionsminimum und einem benachbarten Peak ein gutes Maß für die Konzentration des Analyten im Gewebe. Der Begriff "lokales Minimum" bedeutet hier, dass das Absorptionsvermögen des Analyten bei der gegebenen Wellenlänge kleiner ist als bei nahegelegenen Wellenlängen. Insbesondere beträgt in bevorzugten Ausführungsformen die Absorptionsfähigkeit bei diesen lokalen Minima, die als Analyt-charakteristische Wellenlängen dienen, mehr als 5 %, vorzugsweise mehr als 10 %, noch besser mehr als 20 %, und am besten mehr als 30 % des höchsten Absorptionspeaks, der mit einer der Analyt-charakteristischen Wellenlängen verbunden ist, auf die sich die Analytmessung stützt. Während Wellenlängen, die genau den Absorptionsspitzen oder lokalen Absorptionsminima entsprechen, in vielen Fällen eine gute Wahl für die für den Analyten charakteristischen Wellenlängen sind, können auch Wellenlängen in der Nähe der Maxima/Minima oder zwischen Maxima und Minima verwendet werden. Die Auswahl von geeigneten Wellenlängen oder Wellenlängenbändern kann aus einem kontinuierlichen Spektrum erfolgen und diese Auswahl wird jeweils für einen nachzuweisenden Analyten unter Berücksichtigung der übrigen Stoffe getroffen, die mit dem Analyten vermischt sind und durch ihre spezifischen Absorptionsspektren Störungen erzeugen.

Dementsprechend können unter "Analyt-charakteristischen Wellenlängen" auch grundsätzlich alle Wellenlängen verstanden werden, bei denen ein Analyt überhaupt Strahlung absorbiert. Spezieller können darunter im Folgenden auch selektierte Wellenlängen verstanden werden, bei denen die Absorptionsdifferenz zu der am nächsten liegenden Absorptionsspitze oder dem am nächsten liegenden lokalen Absorptionsminimum weniger als 30 %, vorzugsweise weniger als 20 % der Absorptionsdifferenz zwischen der am nächsten liegenden Absorptionsspitze und dem am nächsten liegenden lokalen Absorptionsminimum beträgt. Zu den für den Analyten charakteristischen Wellenlängen können auch Wellenlängen gehören, bei denen die Absorption anderer Substanzen, mit denen der Analyt im Gewebe vermischt ist, besonders gering ist.

Ferner wird in einigen Anwendungsfällen zum Nachweis der Absorption eine physikalische Reaktion eines Messkörpers oder einer darin enthaltenen Komponente/eines darin enthaltenen Stoffs auf Wärme- und/oder Druckwellen gemessen, die bei Absorption der Anregungsstrahlung aus einer Probe / dem Gewebe empfangen werden. Diese Wärme- und/oder Druckwellen werden dann unter Verwendung einer Detektionsvorrichtung detektiert, die auf der Grundlage der detektierten physikalischen Reaktion ein Antwortsignal erzeugt, wobei das Antwortsignal den Absorptionsgrad der Anregungsstrahlung und damit auch die Konzentration des absorbierenden Stoffes angibt.

Im Folgenden können die Schritte des Einstrahlens einer Anregungsstrahlung in das Gewebe zur Absorption durch den darin enthaltenen Analyten und des Erzeugens eines Antwortsignals, in einigen Fällen aufgrund der Erfassung einer physikalischen Reaktion eines Messkörpers, auch als "Durchführen einer Analytmessung" oder als "Messung einer Stoffkonzentration" bezeichnet werden.

Die vorliegende Erfindung ist weder auf eine bestimmte physikalische Reaktion auf Wärme- und/oder Druckwellen beschränkt, die aus dem Volumen oder der Probe oder dem Gewebe nach Absorption der Anregungsstrahlung empfangen werden, noch auf eine bestimmte Methode zum Erfassen dieser physikalischen Reaktion, die die Erzeugung eines Antwortsignals ermöglicht, das den Absorptionsgrad der Anregungsstrahlung angibt.

Für die anwendbaren Arten von Analytmessverfahren wurden bereits verschiedene physikalische Reaktionen und entsprechende Nachweismethoden vorgeschlagen, die nachfolgend kurz zusammengefasst werden und jeweils bei der vorliegenden Erfindung Anwendung finden können.

Beispielsweise kann die Detektionsvorrichtung einen Messkörper umfassen, der mit der Probe im Kontakt steht und in den Druck- und/oder Wärmewellen von der Probe übertragen werden. Die Detektionseinrichtung kann dann eine Lichtquelle zum Erzeugen eines Detektionslichtstrahls umfassen, der sich durch mindestens einen Teil des Messkörpers oder einer in dem Messkörper enthaltenen Komponente ausbreitet. Die Reaktion des Messkörpers auf Wärme- und/oder Druckwellen, die aus der Probe/ dem Gewebe bei Absorption der Anregungsstrahlung empfangen werden, kann eine lokale Änderung des Brechungsindex des Messkörpers oder der Komponente sein.

In diesem Fall kann die Detektionsvorrichtung zum Detektieren einer Änderung des Lichtwegs oder einer Änderung der Phase des Detektionslichtstrahls aufgrund der Änderung des Brechungsindex des Materials des Messkörpers oder der in diesem enthaltenen Komponente eingerichtet sein.

Beispielsweise ist bei verschiedenen Verfahren und Vorrichtungen, die ausführlich in zwei früheren Anmeldungen beschrieben sind, veröffentlicht als WO 2015/193310 A1 und WO 2017/097824 A1, die beide hierin durch Bezugnahme einbezogen sind, der Messkörper für die Detektion transparent für die Anregungsstrahlung und der Detektionslichtstrahl ist so gerichtet, dass er vollständig oder teilweise an einer Oberfläche des Messkörpers reflektiert wird, die mit dem Gewebe/-der Probe in Kontakt steht.

In diesem Fall kann die Detektionseinrichtung einen Photodetektor umfassen, insbesondere einen positionsempfindlichen Photodetektor, der in der Lage ist, einen Ablenkungsgrad, insbesondere einen Ablenkwinkel, des Detektionslichtstrahls aufgrund der lokalen Änderung des Brechungsindex in dem Messkörper zu detektieren. In diesem Fall ist die physikalische Reaktion auf die vom Messkörper empfangenen Wärme- und/oder Druckwellen eine lokale Änderung des Brechungsindex, und das Antwortsignal ist der erfasste Grad der Ablenkung, der sich tatsächlich als Indikator für den Grad der Absorption der Anregungsstrahlung erweist.

In weiter vorgeschlagenen alternativen Varianten, wie sie beispielsweise in der hierin durch Bezugnahme aufgenommenen internationalen Anmeldung PCT/EP2019/064356 offenbart sind, kann die Detektionsvorrichtung eine interferometrische Vorrichtung in einem Messkörper, beispielsweise ein Ringresonator-Interferometer, umfassen, die die Beurteilung der Phasenänderung des Detektionsstrahls ermöglicht und ein Antwortsignal erzeugt, das die Phasenänderung anzeigt.

In diesem Fall ist die physikalische Reaktion des Messkörpers (oder einer darin enthaltenen Komponente) auf die von der Probe empfangenen Wärme- und/oder Druckwellen eine lokale Änderung des Brechungsindex, während das Antwortsignal in diesem Fall ein interferometrisches Signal ist, das auf einer Änderung der Phase des Detektionsstrahls aufgrund der lokalen Änderung des Brechungsindex basiert.

In noch weiteren möglichen Ausführungsformen kann der Messkörper oder eine Komponente in dem Messkörper elektrische Eigenschaften aufweisen, die sich als Reaktion auf eine lokale Temperaturänderung und/oder eine damit verbundene Druckänderung ändern, und die Erfassungsvorrichtung umfasst Elektroden zum Erfassen elektrischer Signale, die diese variablen Eigenschaften darstellen.

Verschiedene mögliche Aufbauten werden in WO 2019/110597 A2 offenbart, das hiermit durch Bezugnahme aufgenommen wird. Beispielsweise kann der Messkörper Abschnitte mit piezoelektrischen Eigenschaften umfassen und Druckänderungen, die mit der auf den Messkörper übertragenen Wärmewelle und/oder Druckwelle einhergehen, führen zu piezoelektrischen Spannungssignalen, die mit den Elektroden aufgezeichnet werden können. In weiteren Varianten kann eine Temperaturänderung aufgrund der übertragenen Druck- oder Wärmewelle mit sehr empfindlichen Temperatursensoren direkt gemessen werden.

In einigen Beispielen kann der Messkörper oder eine Komponente in dem Messkörper ein Volumen umfassen, in dem sich akustische Wellen, wie etwa Schallwellen, ausbreiten können, beispielsweise einen Hohlraum, eine Aussparung und/oder einen akustischen Resonator, die beispielsweise gasgefüllt sein können.

Die physikalische Reaktion des Messkörpers bzw. dieser Komponente im Messkörper kann somit eine Schallwelle sein, z.B. eine longitudinale Druckwelle in dem Volumen, die durch eine vom Gewebe bei Absorption der Anregungsstrahlung empfangene Wärme- und/oder Druckwelle angeregt wird. Die Erfassungseinrichtung kann ein Mikrofon umfassen, das in dem Volumen angeordnet ist oder mit diesem in Kontakt steht. Das Mikrofon kann als Wandler fungieren, um Schallwellen in elektrische Signale umzuwandeln, wobei die elektrischen Signale das oben genannte Antwortsignal darstellen können, das den Grad der Absorption der Anregungsstrahlung angeben.

Weiter unten wird die physikalische Reaktion des Messkörpers auf die im Gewebe erzeugte Temperaturerhöhung infolge des thermischen Kontakts zwischen dem Messkörper und dem Gewebe detailliert beschrieben.

In verschiedenen Ausführungsformen der Vorrichtung und des Verfahrens der Erfindung steht das Gewebe in druckübertragendem Kontakt mit dem Messkörper und die physikalische Reaktion des Messkörpers ist eine Reaktion auf vom Gewebe empfangene Druckwellen. Der Ausdruck "druckübertragender Kontakt" soll hier alle Beziehungen umfassen, die die Übertragung von Druckwellen vom Gewebe auf den Messkörper ermöglichen, und insbesondere auch eine akustisch gekoppelte Wechselwirkung, bei der die Kopplung durch ein Gas oder eine Flüssigkeit oder einen festen Körper hergestellt werden kann.

Der Begriff "Analytmessverfahren" oder "Analytmessung" weist daraufhin, dass diese Messverfahren auf Antwortsignalen basieren, die mit Anregungsstrahlung bei für den Analyten oder den nachzuweisenden Stoff geeigneten Wellenlängen erhalten werden.

Da das Antwortsignal den Absorptionsgrad der Anregungsstrahlung durch den Analyten oder den Stoff, dessen Konzentration gemessen werden soll, angibt, steht das Antwortsignal in direktem Zusammenhang mit der Konzentration des Analyten im Gewebe oder der Probe/dem Messvolumen.

Dementsprechend basiert der Analyseschritt zumindest teilweise auf einer Konzentration des Analyten im Gewebe und kann in einigen nicht einschränkenden Anwendungen tatsächlich auf die Bestimmung dieser Konzentration hinauslaufen.

Das obige Verfahren wurde beispielsweise bereits in Geräten zur nichtinvasiven Messung des Glukosespiegels eines Benutzers eingesetzt.

Bei dieser speziellen Anwendung besteht der "Analyt" aus Glukose und das "Gewebe" ist die Haut des Benutzers und das Volumen ist das Volumen unterhalb der Hautoberfläche. Es wurde bereits gezeigt, dass mit dieser Methode die Glukosekonzentration in der interstitiellen Flüssigkeit in der Haut eines Menschen sehr präzise gemessen werden kann. Diese Konzentration steht in direktem Zusammenhang mit dem Glukosegehalt im Blut des Patienten und ist daher repräsentativ für diesen. In Fig. 4 der vorliegenden Anmeldung ist das Ergebnis einer Clark'schen Fehlergitteranalyse aus WO 2017/097824 A1 dargestellt und zeigt, dass mit der obigen Analysemethode die tatsächliche Glukosekonzentration einer Person sehr genau vorhergesagt werden kann.

Das obige Verfahren kann in stationären Geräten implementiert werden, die beispielsweise in einer klinischen Umgebung oder im Zuhause eines Patienten zum Überwachen eines Analytspiegels wie etwa eines Glukosespiegels des Benutzers oder Patienten (Probanden) verwendet werden können.

Ein Ziel der vorliegenden Erfindung besteht darin, Mittel bereitzustellen, Anregungsstrahlen mit einer bestimmten Auswahl an Anregungswellenlängen in einfacher und zuverlässiger Weise erzeugen und steuern zu können Weiter sollen Mittel bereitgestellt werden, um den Nachweis und/oder die Messung einer Stoffkonzentration zuverlässiger, schneller und genauer durchführen zu können.

Diese Aufgabe wird durch eine Anregungsquelle sowie eine Vorrichtung zum Nachweis eines Stoffs gemäß den unabhängigen Patentansprüchen gelöst.

Beispiele von möglichen Ausführungsformen sind in den abhängigen Ansprüchen detailliert aufgeführt.

Somit bezieht sich die Erfindung auf eine Anregungsquelle zur Einstrahlung von Anregungsstrahlung in ein Volumen, mit einer Mehrzahl von Anregungsquellenelementen, von denen jedes eine Anregungsstrahlung in einem ihm zugeordneten Wellenlängenbereich in einer gemeinsamen Strahlrichtung aussendet, wobei wenigstens eine erste und eine zweite Gruppe mit jeweils mehreren Anregungsquellenelementen vorgesehen sind, wobei die Anregungsquellenelemente jeder Gruppe auf einer gemeinsamen, quer zur Strahlrichtung, insbesondere senkrecht zur Strahlrichtung, angeordneten Fläche oder Linie angeordnet sind und wobei die verschiedenen Gruppen von Anregungsquellenelementen in Strahlrichtung voneinander beabstandet sind. Mit einer Richtung quer zur Strahlrichtung können dabei beispielsweise auch Winkel zwischen 90 und 80 Grad, weiter auch Winkel zwischen 90 und 70 oder zwischen 90 und 60 Grad mit umfasst werden.

Mit der Mehrzahl von Anregungsquellenelementen kann eine Anregungsstrahlung mit verschiedenen Wellenlängen gleichzeitig oder nacheinander erzeugt und auf das Volumen oder eine Probe/ein Gewebe gerichtet werden.

Der Umstand, dass eine Mehrzahl von Anregungsquellenelementen einer gemeinsamen Gruppe angehören und auf einer Fläche oder Linie angeordnet sind, die, beispielsweise als Gruppe von Kanten- oder Oberflächenemittern ausgeführt, quer oder senkrecht zu der vorgegebenen Strahlrichtung verläuft, bedeutet, dass die Anregungsquellenelemente einer Gruppe in Richtung der Strahlrichtung auf einer gemeinsamen Höhe oder annähernd auf einer gemeinsamen Höhe, das heißt auch in einem gleichen oder annähernd gleichen Abstand von einem zu bestrahlenden Target nebeneinander angeordnet sind. Das bedeutet in vielen Fällen, dass die Anregungsquellenelemente einer Gruppe in Strahlrichtung gegeneinander nicht versetzt sind. Die Anregungsquellenelemente besitzen oft in Strahlrichtung eine Ausdehnung und können an sich nicht vollständig auf einer Linie oder in einer Fläche angeordnet sein, jedoch kann der obige Wortlaut so aufgefasst werden, dass beispielsweise die Strahlung abgebende Fläche aller Anregungsquellenelemente einer Gruppe oder ein anderer Referenzpunkt in einer Fläche oder auf einer Linie liegt, die quer oder senkrecht zur Strahlrichtung liegt.

Dazu kann vorgesehen sein, dass eine Mehrzahl von Anregungsquellenelementen oder alle Anregungsflächenelemente mehrerer Gruppen oder zumindest einer Gruppe jeweils die gleiche Form und/oder Größe aufweisen.

Diese Anordnung hat zur Folge, dass die Anregungsstrahlen der Anregungsquellenelemente einer Gruppe jeweils in einfacher Weise durch ein gemeinsames Strahlformungselement oder durch eine Anzahl gleichartiger Strahlführungselemente geführt, also beispielsweise kollimiert oder fokussiert werden können. Da jedes Anregungsquellenelement in Richtung senkrecht zu seiner Strahlrichtung eine Ausdehnung besitzt, können mehrere Anregungsquellenelemente nebeneinander nur in begrenzter Dichte angeordnet werden. Werden die Anregungsquellenelemente in der Strahlrichtung gegeneinander versetzt, so können alle Anregungsquellenelemente dichter an einer zentralen Achse der Anregungsstrahlen angeordnet werden, die in der gemeinsamen Strahlrichtung verläuft.

Zudem schafft der Versatz von wenigstens zwei Gruppen von Anregungsquellenelementen gegeneinander in Strahlrichtung mehr Möglichkeiten zur Abfuhr von Verlustwärme, als wenn alle Anregungsquellenelemente in Bezug auf die Strahlrichtung auf gleicher Höhe lägen. Es kann somit beispielsweise für jede Gruppe von Anregungsquellenelementen eine eigene Kühlung vorgesehen werden.

Eine besondere Ausführungsform kann zudem vorsehen, dass wenigstens eine erste und eine zweite Gruppe von Anregungsquellenelementen jeweils entlang von senkrecht zur Strahlrichtung verlaufenden Linien angeordnet sind, wobei die Linien dieselbe Form aufweisen und gegeneinander in Richtung senkrecht zur Strahlrichtung versetzt sind.

Somit können mehrere Gruppen von Anregungsquellenelementen gleichartig gestaltet und nach demselben Muster hergestellt und kombiniert werden, um eine möglichst große Anzahl von Anregungsquellenelementen zwar in Strahlrichtung gegeneinander versetzt, jedoch nahe einer zentralen Achse der Strahlrichtung anordnen zu können. Alle durch die Anregungsquellenelemente erzeugten Anregungsstrahlen können damit auf einem relativ engen Raum in der Nähe einer zentralen Achse angeordnet oder konzentriert werden.

Weiter kann vorgesehen sein, dass die Linien zueinander abschnittsweise parallel verlaufen.

Mit dieser Anordnung zweier Linien von Anregungsquellenelementen können diese, in Richtung des Anregungsstrahls betrachtet, besonders nahe zueinander und zu einer zentralen Achse der Anregungsstrahlen angeordnet werden, ohne einander zu überdecken. Damit lassen sich Anregungsstrahlen der verschiedenen Anregungsquellenelemente auch in einfacher Weise auf einer optischen Achse durch gemeinsame optische Strahlführungselemente beeinflussen. Unter der zentralen Achse der Anregungsstrahlen kann beispielsweise eine Achse verstanden werden, von der aus die Summe der Abstände aller Anregungsstrahlen der Anregungsquellenelemente minimiert ist.

Es kann weiter vorgesehen sein, dass die Linien gerade Linien sind.

Die Anordnung von mehreren Anregungsquellenelementen auf einer geraden Linie bietet sich oft an, wenn die Anregungsquellenelemente auf einem Halbleiterwafer gebildet sind, da Halbleiterwafer meistens gerade Konturen aufweisen. Es können somit beispielsweise zwei Halbleiterwafer, von denen jeder eine Gruppe von Anregungsquellenelemente trägt, gegeneinander in Strahlrichtung versetzt miteinander kombiniert werden.

Es kann beispielsweise vorgesehen sein, dass die Anregungsquellenelemente Quantenkaskadenlaser sind.

Quantenkaskadenlaser lassen sich in der Form von Arrays auf einem Halbleiterwafer mit relativ geringem Platzaufwand herstellen. Dabei ist jedoch aus thermischen Gründen und aus Gründen des Herstellungsverfahrens ein Mindestabstand zwischen benachbarten Quantenkaskadenlasern einzuhalten. Dadurch lassen sich in einem linienförmigen Array, das heißt einer Reihe von Lasern, nur relativ wenige Anregungsquellenelemente in der Form von Lasern so unterbringen, dass ihre parallel zueinander ausgesandten Strahlen noch auf einer gemeinsamen optischen Achse gesammelt und durch gemeinsame Makrolinsen geführt werden können. Werden mehr Anregungsquellenelemente benötigt, so kann jedoch eine weitere Reihe gebildet werden. Diese kann oft nicht unmittelbar neben oder über eine erste Reihe gesetzt werden, da die Herstellung schwierig ist und zudem die Temperaturstabilisierung oder Kühlung von vielen Quantenkaskadenlasern auf engem Raum problematisch ist. Für einen solchen Fall ist die Beabstandung der Gruppen von Quantenkaskadenlasern in der Strahlrichtung hilfreich.

Eine weitere Ausführungsform kann beispielsweise vorsehen, dass jede der Gruppen von Anregungsquellenelementen auf jeweils einem separaten Halbleiterwafer angeordnet ist.

Unter einem Halbleiterwafer kann in diesem Zusammenhang ein bereits strukturierter Wafer verstanden werden, der bereits zu einem Chip oder QCL-Element mit mehreren Anregungsquellenelementen weiterverarbeitet ist.

Hierdurch wird die Herstellung der einzelnen Gruppen von Anregungsquellenelementen, insbesondere Quantenkaskadenlasern, einfach und es sind danach nur noch die verschiedenen Wafer zueinander in Position zu bringen.

Es kann zudem vorgesehen sein, dass wenigstens zwei der separaten Halbleiterwafer, auf denen jeweils eine Gruppe von Anregungsquellenelementen angeordnet ist, miteinander fest verbunden sind.

Auch hier kann unter den Halbleiterwafern jeweils ein strukturierter Wafer verstanden werden, der bereits zu einem Chip oder QCL-Element mit mehreren Anregungsquellenelementen weiterverarbeitet ist.

Durch eine feste Verbindung der verschiedenen Halbleiterwafer können die einzelnen Anregungsquellenelemente, insbesondere Quantenkaskadenlaser, die die Anregungsquellenelemente bilden, zuverlässig und dauerhaft zueinander ausgerichtet und positioniert werden. Die verschiedenen Halbleiterwafer können miteinander verklebt oder verlötet werden und sie können jeweils in Epitaxieverfahren gebildete Justageflächen aufweisen, die eine Ausrichtung verschiedener Halbleiterwafer aneinander in einfacher Weise ermöglichen. Die einzelnen Halbleiterwafer, die jeweils eine Gruppe von Anregungsquellenelementen aufweisen, können für sich auch jeweils als Array von Anregungsquellenelementen bezeichnet werden.

Eine weitere Ausführungsform kann darin bestehen, dass eine gemeinsame Ansteuereinrichtung vorgesehen ist, die eine Mehrzahl von Anregungsquellenelementen ansteuert.

Damit kann sowohl die Intensität als auch die Modulation der einzelnen Anregungsquellenelemente und deren Betriebszeit- und Dauer aufeinander abgestimmt gesteuert werden.

Es kann auch vorgesehen sein, dass eine gemeinsame Makrolinse zur Strahlformung der Anregungsstrahlung von mehreren oder allen Anregungsquellenelementen einer Gruppe oder mehrerer verschiedener, in Strahlrichtung voneinander beabstandeter Gruppen vorgesehen ist.

Eine solche Makrolinse ermöglicht die Führung, Fokussierung, Kollimierung oder Bündelung der Anregungsstrahlen einer Vielzahl von Anregungsquellenelementen auf einer gemeinsamen optischen Achse, die in den meisten Fällen entlang der Strahlrichtung verläuft.

Eine weitere Ausführungsform kann darin bestehen, dass für wenigstens eine der Gruppen von Anregungsquellenelementen eine separate Mikrolinsenanordnung vorgesehen ist. Die Mikrolinsenanordnung kann in den meisten Fällen eine Mehrzahl von Mikrolinsen enthalten. Dabei kann vorgesehen sein, dass jede einzelne Mikrolinse der Strahlformung der Anregungsstrahlung von jeweils einem einzelnen Anregungsquellenelement dient.

Die jeweilige Mikrolinse kann dann jeweils vor dem ihr zugeordneten Anregungsquellenelement, das heißt zwischen diesem und einem zu beleuchtenden oder zu bestrahlenden Target, angeordnet sein.

Die Mikrolinsen können in Strahlrichtung sehr nah bei den Anregungsquellenelementen angeordnet sein, so dass die gesamte Anregungsquelle platzsparend hergestellt werden kann und sehr viel von der abgegebenen Strahlung durch die Mikrolinsen gesammelt werden kann. Zudem kann jede der Mikrolinsen genau zentral vor einem Anregungsquellenelement angeordnet und mit seiner optischen Achse auf dieses ausgerichtet sein, so dass Abbildungsfehler minimiert werden können.

Die Anregungsquelle kann derart ausgebildet sein, dass wenigstens einer der Gruppen von Anregungsquellenelementen Mikrolinsen zugeordnet sind, die eine azirkular-zylindrische Form aufweisen und von denen jeweils eine vor einem der Anregungsquellenelemente angeordnet ist.

Die azirkular- zylindrische Form der Mikrolinsen bedeutet, dass diese auf zumindest einer ihrer Seiten eine zylindrische Form, jedoch keinen kreiszylindrischen Querschnitt aufweisen. Hierdurch können Aberrationsfehler verringert werden, die zum Teil beispielsweise auf die große Nähe der Anregungsquellenelemente zu der ihnen jeweils zugeordneten Mikrolinse und den großen Öffnungswinkel des eintretenden Strahlungsbündels zurückzuführen sind.

Es kann auch vorgesehen sein, dass jede der Mikrolinsen zwei voneinander abgewandte, jeweils azirkular-zylindrische Außenflächen aufweist. Die Zylinderachsen der beiden Außenflächen können dabei senkrecht zueinander stehen. Jede der Außenflächen kann speziell auf eine lineare Polarisationsachse der durch die Anregungsquellenelemente erzeugten Strahlung angepasst sein. Da in vielen Fällen die Strahlung in einem Halbleiterelement, beispielsweise einem Halbleiterlaser, weiter beispielsweise in einem Quantenkaskadenlaser erzeugt wird, welche dann das jeweilige Anregungsquellenelement bilden, ist in vielen Fällen ein optisch mehrachsiges Material beteiligt, so dass für die verschiedenen Polarisationsrichtungen unterschiedliche Dielektrizitätskonstanten und Ausbreitungsgeschwindigkeiten gelten. Es kann somit eine schnelle Achse und eine langsame Achse der polarisierten Strahlung vorgesehen sein und die beiden Polarisationsanteile können dann hauptsächlich durch jeweils eine der verschiedenen azirkuar-zylindrischen Aussenflächen der Mikrolinse kollimiert werden.

Eine weitere Ausführungsform kann vorsehen, dass jede Anordnung von Mikrolinsen, die einer Gruppe von Anregungsquellenelementen zugeordnet ist, als Mikrolinsenarray ausgebildet ist, in dem die Mikrolinsen fest miteinander verbunden sind.

Ein solches Mikrolinsenarray kann als eindimensionale Reihe oder als 2-dimensionales Array von Mikrolinsen ausgebildet sein. Die einzelnen Mikrolinsen eines Arrays sind dann herstellungstechnisch, also beispielsweise durch ein Epitaxieverfahren oder eine andere hochpräzise Herstellungsmethode, bereits hochpräzise zueinander positioniert und ausgerichtet. Die Montage der Anregungseinrichtung mit der Anregungsquelle wird dadurch erheblich erleichtert. Die Mikrolinsen können durch ein AR-coating entspiegelt sein, um einen möglichst großen Transmissionsfaktor zu realisieren.

Es kann außerdem vorgesehen sein, dass alle oder wenigstens 80% der Anregungsquellenelemente einer ersten Gruppe eine größere Wellenlänge aufweisen als alle Anregungsquellenelemente einer zweiten Gruppe.

Eine derartige Zuordnung der Wellenlängen zu den einzelnen Anregungsquellenelementen, beispielsweise zu Quantenkaskadenlasern, kann ermöglichen, dass jede Gruppe von Anregungsquellenelementen einen bestimmten zusammenhängenden Wellenlängenbereich abdeckt, wodurch die Ansteuerung vereinfacht werden kann. Zudem besteht ein Vorteil darin, dass das Epitaxieverfahren zur Herstellung der einzelnen Arrays, die jeweils eine Gruppe von Anregungsquellenelementen aufweisen, für einen bestimmten Wellenlängenbereich optimiert werden kann.

Auch die Kühlleistung/Entwärmungsleistung kann dann, während bestimmte Anregungsquellenelemente eines zusammenhängenden Wellenlängenbereichs betrieben werden, auf eine der Gruppen konzentriert werden. Zu diesem Zweck können ein oder mehrere Peltierelemente an den Halbleiterwafern/Halbleiterelementen der Anregungseinrichtung angeordnet sein. Zur Temperaturmessung können beispielsweise NTC-Halbleiterelemente auf den Halbleiterwafern oder in ihrer Nähe vorgesehen sein, so dass die Temperatur der Anregungseinrichtung, zumindest die Temperatur aller oder einer ausgewählten Gruppe der Anregungsquellenelemente, jeweils während der Zeit, in der diese betrieben werden, konstant gehalten werden kann. Durch eine gezielte Temperatursteuerung kann die Wellenlänge der einzelnen Anregungsquellenelemente auch gezielt um einen bestimmten, geringen Betrag verändert werden. Dies erlaubt in manchen Fällen eine Anpassung der Wellenlängen an bestimmte Messbedingungen oder Umgebungsbedingungen.

Die Erfindung bezieht sich außer auf eine Anregungsquelle der oben beschriebenen Art auch auf eine Vorrichtung zum Nachweis eines Stoffes, insbesondere zur Messung einer Stoffkonzentration, in einem Volumen, insbesondere einer Probe oder einem Gewebe, mit einer Anregungsquelle der oben beschriebenen Art zur Einstrahlung einer Anregungsstrahlung verschiedener Wellenlängen in das Volumen und mit einer Detektionseinrichtung zur Erfassung eines durch die Absorption der Strahlung in dem Volumen oder dem Gewebe oder der Probe erzeugten Antwortsignals in Abhängigkeit von der Wellenlänge der Anregungsstrahlung, sowie mit einer Modulationseinrichtung zur Modulation des Verlaufs der Intensität der Anregungsstrahlung.

Durch die Verwendung einer Anregungsquelle der beschriebenen Art lässt sich eine Vorrichtung zum Nachweis eines Stoffes platzsparend herstellen. Die Funktion kann durch die Verwendung von einzelnen Anregungsquellenelementen, denen beispielsweise bestimmte Wellenlängen zugeordnet sind, in vielen Fällen verbessert werden, da beispielsweise ein Durchscannen von Wellenlängen mit einem durchstimmbaren Anregungsquellenelement zu Funktionsschwankungen und zu einer längeren Messzeit führen kann.

Durch die beschriebene geometrische Anordnung der Anregungsquellenelemente in Gruppen, die in Strahlrichtung voneinander beabstandet sind, kann eine ausreichende Anzahl von Anregungsquellenelementen in einer geringen Entfernung von einer optischen Achse der Anregungsquelle angeordnet werden. Dadurch wird eine Strahlformung ermöglicht, die für alle oder möglichst viele der Anregungsquellenelemente zu einem gemeinsamen bestrahlten Spot mit einer kompakten Form führt, die einem Kreis relativ nahekommt oder elliptisch geformt ist. Der Durchmesser eines solchen Bestrahlungsspots kann dabei zwischen 50 und 1000 Mikrometern, insbesondere zwischen 250 und 350 Mikrometern und weiter insbesondere zwischen 270 und 330 Mikrometern liegen.

In diesem Größenbereich kann einerseits eine genügend große Konzentration der Bestrahlungsstärke oder Strahlungsintensität erreicht werden und andererseits ist ein solcher Spot groß genug, um lokal variierende Eigenschaften einer Probenoberfläche, beispielsweise menschlicher Haut, herauszumitteln.

Zudem ist bei Verwendung einer Anregungsquelle der beschriebenen Art für die Anwendung verschiedener Wellenlängen von Strahlung das Messverfahren gegenüber einer durchstimmbaren Strahlungsquelle deutlich beschleunigt, so dass die Messzeit verkürzt werden kann oder zusätzliche Messungen, wie beispielsweise eine wellenlängenabhängige Dämpfungsmessung in der Probe durchgeführt werden kann. Auch können analytspezifische Wellenlängen vorausgewählt und in den Anregungsquellenelementen etabliert werden, beispielsweise die auf die Analyse und Konzentrationsmessung von Glukose in menschlicher Haut optimierten Wellenlängen.

Die beschriebene Anregungsquelle kann vorteilhaft im Rahmen einer Vorrichtung zur Stoffanalyse oder zum Nachweis eines Stoffes unter Verwendung eines Messverfahrens der thermo-optischen Spektroskpie verwendet werden. Dazu kann in an sich bekannter Weise beispielsweise eine Detektionseinrichtung zur Erfassung eines durch die Absorption der Strahlung in dem Volumen oder dem Gewebe oder der Probe erzeugten Antwortsignals verwendet werden, die beispielsweise einen Messkörper der weiter unten noch für verschiedene Messmethoden beschriebenen Art umfasst.

Die erfindungsgemäße Anordnung kann beispielsweise bei einem Verfahren zum Nachweis eines Stoffs, insbesondere zur Ermittlung einer Stoffkonzentration, in einem Volumen, insbesondere in einem Gewebe oder einer Probe eingesetzt werden, bei dem mittels einer Anregungsquelle Anregungsstrahlung verschiedener Wellenlängen in das Volumen eingestrahlt und eine durch die Absorption der Anregungsstrahlung in dem Volumen erzeugte Reaktion des in dem Volumen befindlichen Materials anhand eines infolge der Reaktion erzeugten Antwortsignals in Abhängigkeit von der Wellenlänge der Anregungsstrahlung erfasst wird, wobei die Intensität der Anregungsstrahlung mit einer Modulationscharakteristik moduliert wird.

Dabei können bei der Ermittlung einer Stoffkonzentration für die Anregungsstrahlung bei wenigstens zwei, wenigstens drei, vier, fünf, sieben oder zehn Wellenlängen oder Wellenlängenbereichen Modulationscharakteristiken angewendet werden, die sich für verschiedene Wellenlängen oder Wellenlängenbereiche teilweise oder vollständig unterscheiden. Hierzu können für mehrere oder alle der Wellenlängen der Anregungsstrahlung die jeweils angewendeten Modulationscharakteristiken unter Berücksichtigung der Dämpfung der Anregungsstrahlung in dem Volumen, insbesondere in dem Gewebe, ermittelt werden, indem für jeweils eine Wellenlänge entweder mit mehr als 3 oder mehr als 4 oder mehr als 5 verschiedenen Modulationscharakteristiken, insbesondere mit jeweils mehr als 3 oder mehr als 4 oder mehr als 5 verschiedenen Modulationsfrequenzen, Anregungsstrahlung in das Volumen eingestrahlt und die Antwortsignale erfasst werden oder in-dem Anregungsstrahlung in Form eines oder mehrerer Anregungs-impulse in das Volumen eingestrahlt und die Antwortsignale im Zeitbereich erfasst werden und in beiden Fällen aus den Antwortsignalen ein Verlauf der Dämpfungsfunktion der Anregungsstrahlung in dem Volumen ermittelt wird.

Aus der Dämpfungsfunktion kann weiter die Sättigung der Antwortsignale in Abhängigkeit von den Modulationscharakteristiken oder von den durch die Modulationscharakteristiken bedingten thermischen Diffusionslängen ermittelt werden, das heißt in Abhängigkeit von den Tiefen in dem Volumen, die bei einer Messung bei den einzelnen Modulationscharakteristiken erreicht werden. Darauf können Modulationscharakteristiken ausgewählt werden, die eine ausreichende Messtiefe ermöglichen, ohne dass in diesen Tiefen bereits eine zu weit fortgeschrittene Sättigung erreicht ist.

In diesem Zusammenhang kann unter der zu ermittelnden Stoffkonzentration beispielsweise ein relativer Massenanteil oder Volumenanteil des nachzuweisenden Stoffes an der in dem Volumen vorliegenden Materialmischung verstanden werden oder auch eine Stoffmenge des nachzweisenden Stoffes pro Volumen.

Unter der Dämpfung der Anregungsstrahlung kann ein Dämpfungsverlauf oder eine Dämpfungsfunktion der Anregungsstrahlung in Abhängigkeit von der Tiefe unter der Oberfläche des Volumens verstanden werden, in das die Anregungsstrahlung eingestrahlt wird. Die Dämpfungsfunktion kann durch den in der jeweiligen Tiefe bereits absorbierten Anteil der Anregungsstrahlung repräsentiert sein. Der Dämpfungsfunktion entspricht umgekehrt die Funktion, die in Abhängigkeit von der Tiefe die noch verbleibende, in die jeweilige Tiefe durchdringende Restintensität der Anregungsstrahlung angibt. Mit dieser Funktion hängt direkt der Verlauf der Sättigung zusammen, wobei die Sättigung für eine bestimmte Tiefe angibt, welcher Anteil der Anregungsstrahlung bis zu der jeweiligen Tiefe bereits insgesamt absorbiert worden ist.

Der Übersichtlichkeit halber soll der Fall einer zeitlich periodischen Modulation der Intensität der Anregungsstrahlung betrachtet werden. Innerhalb einer Periode der Modulation gelangen jeweils Antwortsignale aus einem bestimmten Tiefenbereich des Volumens, in das die Anregungsstrahlung eingestrahlt wird und in dem die Stoffkonzentration gemessen werden soll, bis an die Oberfläche des Volumens oder des Gewebes. Je größer die Periodendauer der Modulation, also, je geringer die Modulationsfrequenz ist, umso größer ist die Tiefe im Messvolumen unterhalb der Oberfläche, aus der die Antwortsignale, beispielsweise Druckwellen oder Temperatursignale innerhalb der Periode an die Oberfläche gelangen und gemessen werden können. Somit lässt sich die maximale Messtiefe durch die Wahl der Modulationsfrequenz bestimmen. Für die Intensität der gemessenen Antwortsignale spielt zusätzlich die Dämpfung der Anregungsstrahlung innerhalb des Messvolumens/Gewebes eine wichtige Rolle. Je stärker die Anregungsstrahlung bei Erreichen einer bestimmten Tiefe unter der Oberfläche bereits durch Absorption reduziert ist, umso geringer ist die nachweisbare Reaktion in dieser Tiefe.

Wird eine sehr geringe Modulationsfrequenz gewählt, so wird praktisch die Reaktion auf die gesamte Anregungsstrahlung im gesamten Messvolumen nachgewiesen und das erfasste Signal ist gesättigt und unabhängig von der Tiefe, in der die Anregungsstrahlung absorbiert wurde und somit auch unabhängig von der Konzentration des nachzuweisenden Stoffes in dem Volumen. Es ist daher sinnvoll, eine Frequenz der Modulation zu wählen, die einer maximale Tiefe der nachgewiesenen Reaktionen entspricht, die um einen deutlichen Betrag geringer ist als die Tiefe, in der die Sättigung erreicht ist Beispielsweise kann die Modulationsfrequenz, nachdem die Dämpfungsfunktion in dem Volumen ermittelt wurde, so gewählt werden, dass die maximale Tiefe, aus der innerhalb der Periode der Modulation Antwortsignale an die Oberfläche gelangen, so bemessen ist, dass bis zu dieser Tiefe nicht mehr als 70% oder nicht mehr als 80% oder nicht mehr als 90% der Anregungsstrahlung absorbiert sind.

Die Dämpfungsfunktion, das heißt der Verlauf der verbleibenden Intensität der Anregungsstrahlung in Abhängigkeit von der Tiefe unter der Oberfläche des Volumens/Gewebes, ist durch den Absorptionskoeffizienten des Materials in dem Volumen bestimmt. Dieser Absorptionskoeffizient ist abhängig von der Wellenlänge der Anregungsstrahlung. Zudem liegt oft in dem Volumen eine Kombination von übereinanderliegenden Schichten vor, die jeweils verschiedene Absorptionskoeffizienten aufweisen. Die Dämpfung ergibt sich somit aus einer Kombination von Schichtdicken und Absorptionskoeffizienten der Schichten. Wird beispielsweise in der menschlichen Haut gemessen, so hängt die Dämpfungsfunktion von der Dicke der verschiedenen Hautschichten und ihren individuellen Absorptionskoeffizienten ab. Die oberste Hautschicht, das stratum corneum, weist beispielsweise auch in Abhängigkeit von ihrem individuellen Wassergehalt, einen relativ hohen Absorptionskoeffizienten auf, der zudem durch einen variierenden Wassergehalt auch stark variieren kann. Um die wellenlängenabhängigen Modulationscharakteristiken zu ermitteln, kann deshalb zunächst der Dämpfungsverlauf der Anregungsstrahlung bei der jeweiligen Wellenlänge in Abhängigkeit von der Tiefe im Volumen/Messvolumen ermittelt werden.

Zu diesem Zweck wird die Dämpfung an einer bestimmten Anzahl von Stützstellen, das heißt in verschiedenen Tiefen des Volumens, gemessen, die durch bestimmte Modulationscharakteristiken, beispielsweise bestimmte Modulationsfrequenzen gegeben sind. Mit diesen Stützstellen kann die Dämpfungsfunktion ermittelt und danach können unter Berücksichtigung der Dämpfungsfunktion die für die eigentliche Messung der Stoffkonzentration möglichen oder optimierten Modulationscharakteristiken/Modulationsfrequenzen festgelegt werden.

Es wurde gefunden, dass in manchen Fällen mehr als 3, mehr als 4, mehr als 5 oder mehr als 7 verschiedene Modulationscharakteristiken ausreichen, um die Dämpfungsfunktion ausreichend zu charakterisieren.

Eine solche Charakterisierung der Dämpfungsfunktion gelingt jedoch nicht nur unter Verwendung von periodischen Modulationen, sondern auch mit anderen Modulationscharakteristiken, beispielsweise impulsförmigen Modulationen, da diesen im Frequenzraum durch eine Fourier - Transformation bestimmte Überlagerungen von Modulationsfrequenzen zugeordnet werden können.

Unter einer Modulationscharakteristik wird somit zunächst im weitesten Sinne eine zeitliche Profilierung der Intensität der Anregungsstrahlung verstanden, die beispielsweise als eine periodische Modulation oder die Einprägung eines nicht periodischen Intensitätsverlaufs gestaltet sein kann. Im Fall eines nicht periodischen Zeitverlaufs kann die Messung beispielsweise in der Zeitdomäne ausgewertet werden.

Es kann in einer Ausführungsform des beschriebenen Verfahrens vorgesehen sein, dass bei der Messung zur Ermittlung des Verlaufs der Sättigung der Antwortsignale mehr als 50%, insbesondere mehr als 80%, weiter insbesondere alle angewendeten Modulations-frequenzen oberhalb von 47 Hz oder zwischen 1kHz und 3 kHz liegen.

Die Dämpfung der Anregungsstrahlung in der Probe ist, wie oben ausgeführt, einerseits durch die Dicke von wellenlängenabhängig besonders stark oder schwach absorbierenden oberflächennahen Schichten des Volumens/der Probe/des Gewebes und durch den dort individuell geltenden Absorptionskoeffizienten bestimmt, beispielsweise bei einer Messung an der Haut eines Menschen durch die Dicke des Stratum Corneum und dessen Wassergehalt.

Die Dämpfungsfunktion kann beispielsweise unter Berücksichtigung von bekannten Größen wie dem Material der Probe, bei der Messung an der Haut eines Menschen unter Berücksichtigung des Alters, der Hautfarbe, der Hautfeuchtigkeit und anderer Parameter, aus Erfahrungswerten geschätzt werden. Dabei können auch Modelle der Künstlichen Intelligenz eingesetzt werden. Genauer ist die Dämpfungsfunktion jedoch durch das oben beschriebene Verfahren ermittelbar. Die erwähnten Größen, wie das Alter, die Hautfarbe und die Hautfeuchtigkeit, die auf andere Weise ermittelbar sind, können zur Ergänzung oder Plausibilisierung der ermittelten Dämpfungsfunktion herangezogen werden. Da das Stratum Corneum bei der Messung an menschlicher Haut oft einen erheblichen Teil der gesamten Dämpfung ausmacht, können wesentliche Informationen über die Dämpfungsfunktion bereits durch eine Messung in den oberen Hautschichten gewonnen werden. Werden also zur Bestimmung der Dämpfungsfunktion Modulationscharakteristiken/Modulationsfrequenzen gewählt, die einer thermischen Diffusionslänge entsprechen, die etwa der Dicke des Stratum Corneum entsprechen, so kann der Wassergehalt dieser Schicht bestimmt und zusammen mit einer typischen Dicke dieser Hautschicht berücksichtigt werden. Werden Modulationscharakteristiken hinzugenommen, die zu einer Tiefe der Messung führen, die über die Dicke des Stratum Corneum zumindest ein Stück weit hinausgehen, so kann auch die Dicke des Stratum Corneum bestimmt werden und damit eine weitere wichtige Größe zur Charakterisierung der gesamten Dämpfungsfunktion. Eine solche Auswahl der Modulationscharakteristiken kann im Idealfall bereits ausreichen, um die gesamte Dämpfungsfunktion zu extrapolieren. Zur Extrapolation können zusätzlich Erfahrungswerte einbezogen werden. Die Dämpfungsfunktion kann auch auf der Basis der Messungen durch ein entsprechend trainiertes selbstlernendes System bestimmt werden. Nach der Bestimmung der Dämpfungsfunktion können dann die geeigneten oder optimalen Modulationscharakteristiken/Modulationsfrequenzen für den Nachweis des Stoffes und die Messung der Stoffkonzentration in dem Volumen/in der Probe/dem Gewebe so bestimmt werden, dass die maximale Messtiefe noch nicht im Bereich der Sättigung liegt, jedoch deutlich unterhalb der Tiefe, die den Grenzen des Stratum Corneum entspricht. Das Stratum Corneum enthält, da es nicht oder nur minimal am Stoffwechsel beteiligt ist, keine Informationen über aktuelle Stoffkonzentrationen des lebenden Gewebes. Die Messgrößen aus dem Stratum Corneum werden deshalb als Störgrößen betrachtet, deren Einfluss durch geeignete Verarbeitung der Messgrößen in an sich bekannter Weise eliminiert werden kann.

Hierzu werden, wie bei bereits bekannten Messverfahren, bei jeweils einer Wellenlänge Messungen mit mehreren verschiedenen Modulationscharakteristiken durchgeführt, um in einem ersten Fall durch wenigstens eine Modulationscharakteristik, die einer geringeren thermischen Diffusionslänge in der Probe entspricht, einen höheren Signalanteil aus oberflächennahen Schichten des Volumens der Probe zu gewinnen und um in einem zweiten Fall durch wenigstens eine Modulationscharakteristik, die einer größeren thermischen Diffusionslänge in der Probe entspricht, einen höheren Signalanteil aus tieferen Schichten zu gewinnen. Die Unterschiede zwischen den oberflächennahen Messungen und den Messungen mit einem Schwerpunkt in größeren Tiefen kann zur Subtraktion der Effekte der oberflächennahen toten Hautschichten von der Tiefenmessung oder zumindest zur Berücksichtigung der Effekte der oberflächennahen toten Hautschichten bei der Auswertung von Tiefenmessungen durch eine geeignete Verknüpfung der Messwerte dienen. Wenn bei der Messung jeweils für eine Wellenlänge oder einen Wellenlängenbereich der Anregungsstrahlung in verschiedenen Messdurchgängen zwei oder mehr verschiedene Modulationscharakteristiken angewendet werden, kann es gemäß dem erfindungsgemäßen Verfahren jedoch vorteilhaft vorgesehen sein, dass jeweils die Modulationscharakteristiken verschiedener Wellenlängen, die die für die jeweilige Wellenlänge längsten thermischen Diffusionslängen in der Probe erzeugen, sich untereinander unterscheiden. Dies werden im Normalfall bei periodischen Modulationscharakteristiken für jede Wellenlänge jeweils diejenigen Modulationscharakteristiken sein, die die niedrigste Frequenz aufweisen. Werden jeweils bei einer Wellenlänge mehr als zwei verschiedene Modulationscharakteristiken angewendet, dann können auch die jeweils zwei oder mehr als zwei Modulationscharakteristiken, die zu den größten Diffusionslängen in der Probe führen, für verschiedene Wellenlängen verschieden sein. Dabei können diese sich unterscheidenden Modulationscharakteristiken jeweils so gestaltet werden, dass bei Wellenlängen, bei denen eine stärkere Dämpfung der Anregungsstrahlung in der Probe stattfindet, jeweils bei Anwendung mehrerer Modulationscharakteristiken pro Wellenlänge diejenigen Modulationscharakteristiken, die eine längere Diffusionslänge erzeugen (die sogenannten Tiefenmessungen), tendenziell so verändert werden, dass die Diffusionslängen sich verkürzen. Dabei wird die Diffusionslänge selbstverständlich nicht soweit verkürzt, dass sie der Diffusionslänge derjenigen Modulationscharakteristik entspricht, mit der die oberflächennahe Messung ausgeführt wird. Demgemäß werden bei periodischen Modulationen die Frequenzen der Tiefenmessungen, die jeweils geringer sind als die der Oberflächenmessungen, etwas erhöht, um die thermische Diffusionslänge zu verkürzen. Liegen die thermischen Diffusionslängen bei den Tiefenmessungen ohnehin schon deutlich unter der Eindringtiefe der Anregungsstrahlung in das Gewebe, so kann es sinnvoll sein, falls für eine Wellenlänge oder Wellenzahl eine stärkere Dämpfung ermittelt wird, die thermische Diffusionslänge der Tiefenmessung für diese Wellenlänge gegenüber Wellenlängen, bei denen eine geringere Dämpfung ermittelt wird, etwas zu erhöhen, um noch möglichst thermische Signale aus einer größeren Tiefe der Probe/des Gewebes mit aufzusummieren, ohne dass jedoch die thermische Diffusionslänge so groß werden darf, dass alle Absorptionsvorgänge im Gewebe noch thermisch an der Oberfläche nachgewiesen werden. In diesem Fall läge eine sogenannte Sättigung vor, bei der die thermische Antwort nicht mehr davon abhängt, in welcher Tiefe des Gewebes/der Probe die Absorption stattfindet und unabhängig von der Konzentration des Analyten die vollständige Absorption der Anregungsstrahlung in Form von thermischen Signalen an der Oberfläche nachgewiesen wird. Die sich unterscheidenden Modulationscharakteristika für die Tiefenmessungen können nach einer zusätzlichen möglichen Maßgabe so gewählt werden, dass sie zu thermischen Diffusionslängen in der Probe führen, die größer sind als die Dicke des Stratum Corneum. Jedoch sollten die thermischen Diffusionslängen geringer sein als die Eindringtiefe der Anregungsstrahlung in das Gewebe.

Es kann somit generell vorgesehen sein, dass, wenn für einige oder alle Wellenlängen der Anregungsstrahlung, bei denen die Messung zur Ermittlung der Stoffkonzentration durchgeführt wird, jeweils wenigstens zwei Modulationscharakteristiken angewendet werden, zumindest die Modulationscharakteristiken, die für jeweils eine Wellenlänge zu der längsten thermischen Diffusionslänge führen (dies entspricht den Tiefenmessungen im Gegensatz zu den oberflächennahen Messungen), sich wenigstens teilweise für die verschiedenen Wellenlängen untereinander unterscheiden.

Es kann auch, wenn für einige oder alle Wellenlängen/Wellenzahlen mehr als zwei verschiedene Modulationscharakteristiken angewendet werden, vorgesehen sein, dass jeweils für zwei Wellenzahlen die Modulationscharakteristiken, die zu den beiden längsten thermischen Diffusionslängen führen, verschieden sind.

Dabei können die Modulationscharakteristiken, die für die verschiedenen Wellenlängen/Wellenzahlen für die oberflächennahen Messungen verwendet werden, also die jeweiligen Messungen mit geringerer thermischer Diffusionslänge, für alle oder einige Wellenlängen/Wellenzahlen unabhängig von der wellenlängenabhängigen Dämpfung gleich sein, da bei den kurzen thermischen Diffusionslängen, die für diese Messungen bei entsprechend hohen Modulationsfrequenzen erzielt werden, die Dämpfung der Anregungsstrahlung keinen großen Einfluss hat, weil die thermischen Signale, die die Oberfläche des Gewebes/der Probe erreichen, ohnehin weit überwiegend aus oberflächennahen Schichten stammen. Eine Änderung der Dämpfung in gewissen Grenzen ändert hieran nichts. Mögliche Modulationsfrequenzen für diese oberflächennahen Messungen können über 47 Hz, insbesondere über 1khz oder über 1,3 khz liegen.

Bei den Messungen mit niedrigeren Modulationsfrequenzen und entsprechend längeren thermischen Diffusionslängen, bei denen auch die thermischen Wellen aus größerer Tiefe des Gewebes die Oberfläche erreichen, beeinflusst die wellenlängenabhängige Dämpfung die Messergebnisse stärker, da je nach Dämpfung die Anregungsstrahlung tiefer oder weniger tief in das Gewebe hineinreicht. Es muss für diese Messungen eine optimale Auswahl der Diffusionslänge getroffen werden, die von mehreren Parametern abhängt. Dabei spielt unter anderem eine Rolle, dass bei einer zu lang gewählten Diffusionslänge alle thermischen Wellen aus allen Tiefen des Gewebes die Oberfläche erreichen und damit die in Form von Anregungsstrahlung in das Gewebe eingestrahlte Energie, die summiert über die Tiefe vollständig absorbiert wird, auch über die gesamte Tiefe im Gewebe thermische Reaktionen erzeugt, die alle an der Oberfläche nachweisbar werden. Damit spielt es jedoch keine Rolle mehr, in welcher Tiefe die Absorption stattfindet und die Summe der an der Oberfläche nachweisbaren thermischen Reaktionen ist unabhängig von der Konzentration des absorbierenden Stoffs. Diese Parameterkonstellation wird mit "Sättigung" bezeichnet. Eine zu kurz gewählte Diffusionslänge führt dazu, dass die Konzentration in den oberflächennahen Bereichen zu stark gewichtet wird, die zumindest teilweise eine Störgröße darstellt und nichts oder wenig zu der variablen Stoffkonzentration beiträgt, die beim Beispiel der menschlichen Haut beispielsweise in der interstitiellen Schicht gemessen wird. In vielen Fällen wird eine größere nachgewiesene Dämpfung dazu führen, dass eine Modulationscharakteristik gewählt wird, die zu einer größeren Diffusionslänge führt, ohne jedoch zur "Sättigung" zu führen. Es gibt einen relativ breiten Übergangsbereich zwischen einem absolut nichtgesättigten Bereich und einem absolut gesättigten Bereich, in dem komfortabel gemessen werden kann.

Es kann für verschiedene gemessene Dämpfungen oder Dämpfungsfunktionen eine Zuordnungsfunktion oder Zuordnungstabelle gebildet werden, die den für die Wellenlängen/Wellenzahlen bestimmten Dämpfungswerten oder Dämpfungsfunktionen bestimmte Modulationsfrequenzen bei der Messung der Stoffkonzentration für die jeweilige Messung mit der größeren thermischen Diffusionslänge oder die jeweiligen Messungen mit den größeren thermischen Diffusionslängen zuordnet. Diese Zuordnung kann außer von den gemessenen oder bestimmten Dämpfungswerten auch noch von anderen Parametern wie vom Wassergehalt der Haut oder einem Lactatgehalt oder weiteren Parametern abhängen. Im einfachsten Fall kann die Zuordnungsfunktion oder Zuordnungstabelle dadurch erzeugt werden, dass jeweils bei einer Wellenlänge mehrere Messungen jeweils mit derselben kürzeren thermischen Diffusionslänge und unterschiedlichen längeren thermischen Diffusionslängen durchgeführt und im Rahmen einer Kalibrierung die Messergebnisse mit Messergebnissen verglichen werden, die mit einer anderen Messmethode, beispielsweise chemisch, ermittelt worden sind. Eine Kalibrierung kann auch durch Verwendung von Werten erfolgen, die durch eine Simulation mit einem erprobten physikalischen Modell gewonnen sind. Dabei sollte insbesondere das jeweils erreichte Signal/Rausch-Verhältnis verglichen und diejenige Modulationscharakteristik gewählt werden, die zu dem besten Signal/-Rausch-Verhältnis führt.

Eine weitere Ausführungsform kann vorsehen, dass eine Messung der Dämpfung der Anregungsstrahlung in den Bereichen des Volumens, insbesondere des Gewebes, vorgenommen wird, die der Oberfläche, durch die die Anregungsstrahlung in die Probe eingestrahlt wird, am nächsten sind, insbesondere zwischen der Oberfläche und einer Tiefe von 0,1mm oder zwischen der Oberfläche und einer Tiefe von 0,03mm.

Dieser Tiefenbereich reicht in vielen Fällen etwas über die Dicke des stratum corneum hinaus, so dass die Dämpfungsfunktion in diesem Bereich damit berücksichtigt werden und der weitere Verlauf der Dämpfungsfunktion in vielen Fällen geschätzt werden kann.

Es kann dabei vorgesehen sein, dass zur Ermittlung der wellenlängenabhängigen Dämpfung der Anregungsstrahlung in dem Volumen/der Probe ein Test-Absorptionsspektrum des Materials in dem Volumen, insbesondere der Probe oder dem Gewebe, aufgrund einer Messung der Absorption von modulierter Anregungsstrahlung bei einer oder mehreren ausgewählten Wellenlängen ermittelt wird, wobei die Absorption aufgrund einer Reaktion des in dem Volumen befindlichen Materials bei der Absorption ermittelt wird und wobei Test-Modulationscharakteristiken der Anregungsstrahlung verwendet werden, die zu einer kleineren thermischen Diffusionslänge führen als die für die Messung der Stoffkonzentration angewendeten Modulationscharakteristiken.

Somit kann für die Ermittlung der Dämpfung eine sehr ähnliche der dieselbe Messmethodik eingesetzt werden, wie für die Nachfolgende Messung einer Stoffkonzentration.

Es kann somit vorgesehen sein, dass die Messung der Absorption der Anregungsstrahlung bei einer Wellenlänge umfasst, dass mittels der Anregungsquelle nacheinander eine gemäß mehreren verschiedenen Modulationscharakteristiken modulierte Anregungsstrahlung der Wellenlänge in das Volumen, insbesondere das Gewebe, eingestrahlt und eine durch die Absorption der Strahlung in dem Volumen/dem Gewebe erzeugte Reaktion mittels eines durch die Reaktion erzeugten Antwortsignals außerhalb des Volumens erfasst wird.

Für die Erfassung von Antwortsignalen können alle bereits weiter oben dargestellten Messverfahren angewendet werden. Es kann bei einer Dämpfungsmessung an menschlicher Haut beispielsweise eine Modulationscharakteristik gewählt werden, die zu einer thermischen Diffusionslänge in dem Gewebe führt, die zwischen dem 0,5-fachen und dem 1,5- fachen der typischen oder durchschnittlichen Dicke der Hornhaut/des stratum corneum bei einem Menschen. Weiter kann eine Modulationscharakteristik gewählt werden, die zu einer thermischen Diffusionslänge in dem Gewebe führt, die kleiner ist als die typische oder durchschnittliche Dicke der Hornhaut/des stratum corneum bei einem Menschen.

Eine weitere Ausführungsform kann, wie weiter oben bereits angedeutet, vorsehen, dass eine Modulationscharakteristik der Anregungsstrahlung die Form eines zeitlichen Intensitätsverlaufs der Anregungsstrahlung, insbesondere die Form einer Anstiegs- und/oder Abfallfunktion der Intensität oder die Form eines periodischen Intensitätsverlaufs, weiter insbesondere die Form eines rechteck- sinus- oder sägezahnförmigen Intensitätsverlaufs hat, wobei die Modulationscharakteristik insbesondere auch die Bemessung des duty cycle, auch Tastverhältnis genannt, umfasst.

Auch durch die Bemessung des duty cycle, also des Anteils der periodischen Aktivitätsfunktion der Anregungsstrahlung, die der Impulsdauer der Anregungsstrahlung entspricht, kann die Ausbreitung der Wärme- oder Druckwelle in dem Probekörper beeinflusst werden. Wird der duty cycle bei gleicher Frequenz der Modulation kürzer gewählt, so sind die Ruhephasen zwischen den Impulsen der Anregungsstrahlung länger und die Wärme- oder Druckwelle kann sich weiter ungestört von einem nachfolgenden Impuls ausbreiten, wodurch die thermische Diffusionslänge größer wird.

Es kann auch vorgesehen sein, dass die Anregungsstrahlung im Bereich der Wellenlängen der nahen Infrarotstrahlung zwischen 1 Mikrometer und 3 Mikrometern oder im Bereich der mittleren Infrarotstrahlung zwischen 8 Mikrometern und 10,5 Mikrometern liegt.

Es hat sich gezeigt, dass für viele Moleküle, deren Konzentration für biologische oder medizinische Zwecke interessant ist, signifikante und aussagekräftige Absorptionsbereiche in diesem Wellenlängenbereich liegen. Dieser Bereich deckt beispielsweise bei vielen komplexen Molekülen die Frequenzen von bestimmten Eigenschwingungen der Moleküle ab.

Ebenfalls kann generell vorgesehen sein, dass die Absorption von Anregungsstrahlung in dem Volumen durch die Messung einer Temperaturerhöhung des in dem Volumen befindlichen Materials, insbesondere Gewebes, insbesondere durch ein Messverfahren der thermo-optischen oder thermo-akustischen Spektroskopie, gemessen wird.

Diese Messmethoden umfassen die weiter oben konkret genannten Beispiele und sollen auch weitere Messmethoden umfassen, die gegebenenfalls dort nicht explizit genannt sind.

Es kann somit vorgesehen sein, dass zur Messung der Absorption der Anregungsstrahlung in dem Volumen von außerhalb des Volumens eine Messung eines Antwortsignals in Form einer Temperaturänderung oder einer Druckwelle an der Oberfläche des Volumens durch eine akustische Signalerfassung oder eine Messung einer Änderung eines Brechungsindex in einem mit dem in dem Volumen befindlichen Material im Kontakt stehenden Messkörper, durch eine pyrometrische Messung oder durch eine piezoelektrische oder eine interferometrische Messung in einem mit dem in dem Volumen befindlichen Material im Kontakt stehenden Messkörper erfolgt. Gerade bei Verwendung einer pyrometrischen oder interferometrischen Messmethode können die Vorteile eines Arrays aus Anregungsquellenelementen der oben beschriebenen Art voll zur Geltung gebracht werden, da diese Messmethoden, ebenso wie ein Array, in extrem kleinen Bauformen hergestellt werden können und sich somit beispielsweise beim Einbau in ein am Körper getragenes Wearable ergänzen können.

Der Begriff der pyrometrischen Sensoren schließt dabei grundsätzlich jede Art von pyrometrischen Sensoren mit ein, beispielsweise Quotientenpyrometer, Gesamtstrahlungspyrometer, Bolometer, pyroelektrische Sensoren sowie Thermosäulen aus Thermoelementen, Thermographiekameras, Halbleitersensoren und quantenmechanische Photonensensoren, Einzelphotonendetektoren wie z.B. Single-Photon Avalanche Dioden (SPAD), Si -Single-Photon Avalanche Dioden, InGaAs- Avalanche Dioden, Transition Edge Sensor (TES) Detektoren, sowie supraleitende Nanodraht-Einzelphotonendetektoren (SNSPD). Es kann zur pyrometrischen Temperaturmessung auch ein Quanten-Kaskadenlaser oder ein Interband- Kaskadenlaser im reversen Betrieb genutzt werden, in dem er bei Bestrahlung mit Photonen einen Strom liefert, der als Maß des Photonenstroms und damit der Temperatur des strahlenden Mediums, gemessen werden kann. Eine solche Messung kann mit einem Interband- Kaskadenlaser beispielsweise auf eine Wellenlänge eingegrenzt werden, die sich ausreichend von der Schwarzkörperstrahlung bei Raumtemperatur mit einem Strahlungsmaximum bei etwa 10 Mikrometer unterscheidet. Dies gelingt beispielsweise, indem man den Interband-Kaskadenlaser auf einen Nachweis im Bereich der Wellenlänge von 6 Mikrometer einschränkt.

Die interferometrische Messung kann beispielsweise mit einem Ringresonator- Interferometer erfolgen.

Zudem kann vorgesehen sein, dass gleichzeitig oder nacheinander oder zeitlich überlappend mittels einer Anregungsquelle in Form eines Arrays mit mehreren Strahlungselementen Anregungsstrahlung verschiedener Wellenlängen oder Wellenlängenbereiche mit je einer Modulationscharakteristik moduliert in das Volumen eingestrahlt und jeweils ein Antwortsignal erfasst wird.

Es soll somit nicht nur die Erzeugung von Anregungsstrahlung verschiedener Wellenlängen nacheinander, sondern auch möglicherweise gleichzeitig oder überlappend, umfasst sein, wobei für verschiedene Wellenlängen unterschiedliche Modulationscharakteristiken verwendet werden können, um die Antwortsignale auseinanderzuhalten. Damit kann beispielsweise die Messzeit verkürzt werden.

Eine weitere Ausführungsform kann vorsehen, dass nach der Ermittlung von geeigneten Modulationscharakteristiken zur Messung der Stoffkonzentration für mehrere oder alle Wellenlängen oder Wellenlängenbereiche, für die die Absorption in dem Volumen oder einer Probe gemessen wird, je eine Absorptionsmessung mit einer ersten und mit wenigstens einer zweiten Modulationscharakteristik durchgeführt wird und die Messergebnisse jeweils für eine Wellenlänge und für verschiedene Modulationscharakteristiken miteinander verknüpft werden, wobei insbesondere die thermischen Diffusionslängen des in dem Volumen/der Probe befindlichen Materials jeweils für die erste und die zweite Modulationscharakteristik verschieden sind.

Somit können beispielsweise die Messergebnisse, die bei kürzeren Diffusionslängen erhalten werden, von den Messergebnissen, die bei größeren Diffusionslängen erhalten werden, abgezogen oder nach einer geeigneten Gewichtung abgezogen werden, um die Messergebnisse für tiefere Schichten des Volumens/der Probe zu erhalten. Dies ist besonders bei der Messung an menschlicher Haut wichtig, da in den obersten Hautschichten nur Stoffkonzentrationen, bei der Messung von Glukose beispielsweise Glukosekonzentrationen, ermittelt werden, die in den toten Hautschichten vorherrschen und die deshalb zu einem momentanen, sich ständig ändernden Glukosewert keine Information enthalten. Diese Anteile sind damit reine Störgrößen und sollen eliminiert werden.

Die Modulationscharakteristiken, die für die verschiedenen Wellenlängen jeweils zu den längeren Diffusionslängen führen und somit einen Messwert in größeren Gewebetiefen repräsentieren, beispielsweise in der interstitiellen Schicht der menschlichen Haut, sind dabei zumindest für einige Wellenlängen oder Wellenlängenbereiche verschieden.

Die Erfindung bezieht sich außer auf ein Verfahren der oben beschriebenen Art auch auf eine Vorrichtung zum Nachweis eines Stoffes, insbesondere zur Messung einer Stoffkonzentration, in einem Volumen, insbesondere einer Probe oder einem Gewebe, mit einer Anregungsquelle zur Einstrahlung einer Anregungsstrahlung verschiedener Wellenlängen in das Volumen und mit einer Detektionseinrichtung zur Erfassung eines durch die Absorption der Strahlung in dem Volumen oder dem Gewebe oder der Probe erzeugten Antwortsignals in Abhängigkeit von der Wellenlänge der Anregungsstrahlung, sowie mit einer Modulationseinrichtung zur Modulation des Verlaufs der Intensität der Anregungsstrahlung, wobei vorgesehen ist, dass die Vorrichtung dazu eingerichtet ist, beim Nachweis des Stoffes oder der Messung der Stoffkonzentration für wenigstens zwei oder wenigstens drei, vier, fünf, sieben oder zehn Wellenlängen oder Wellenlängenbereiche der Anregungsstrahlung Modulationscharakteristiken anzuwenden, die sich für verschiedene Wellenlängen oder Wellenlängenbereiche wenigstens teilweise unterscheiden und wobei weiter vorgesehen ist, dass die Vorrichtung dazu eingerichtet ist, für mehrere oder alle der Wellenlängen der Anregungsstrahlung die jeweils angewendeten Modulationscharakteristiken unter Berücksichtigung der Dämpfung der Anregungsstrahlung in dem Volumen, insbesondere in dem Gewebe, zu ermitteln, indem für jeweils eine Wellenlänge entweder mit mehr als 3 oder mehr als 4 oder mehr als 5 verschiedenen Modulationscharakteristiken, insbesondere mit jeweils mehr als 3 oder mehr als 4 oder mehr als 5 verschiedenen Modulations-frequenzen, Anregungsstrahlung in das Volumen eingestrahlt und die Antwortsignale er-fasst werden oder indem Anregungsstrahlung in Form eines oder mehrerer Anregungs-impulse in das Volumen eingestrahlt und die Antwortsignale im Zeitbereich erfasst wer-den und die Vorrichtung in beiden Fällen aus den Antwortsignalen einen Verlauf der Sättigung der Antwortsignale in Abhängigkeit von den Modulationscharakteristiken ermittelt.

Bei einer derartigen Vorrichtung kann auch vorgesehen sein, dass sie eine Einrichtung zur Ermittlung der Verläufe der Sättigung der Antwortsignale der Anregungsstrahlung in dem Volumen oder Gewebe oder einer Probe in Abhängigkeit von der Wellenlänge der Anregungsstrahlung aufweist sowie eine Zuordnungseinrichtung, die den ermittelten Verläufen der Sättigung der Antwortsignale in Abhängigkeit von den Modulationscharakteristiken für die einzelnen Wellenlängen und/oder Wellenlängenbereiche jeweils wenigstens eine Modulationscharakteristik und/oder Strahlungsintensität zuordnet, wobei sich die zugeordneten Modulationscharakteristiken und/oder Strahlungsintensitäten für verschiedene Wellenlängen oder Wellenlängenbereiche wenigstens teilweise unterscheiden.

Ein weiteres Ziel der vorliegenden Erfindung besteht darin, Mittel bereitzustellen, um mit einem Verfahren und einer Vorrichtung den Nachweis und/oder die Messung einer Stoffkonzentration schneller, zuverlässiger und genauer durchführen zu können.

Dies kann erreicht werden durch eine Vorrichtung der oben beschriebenen Art zur Erfassung eines Analyten oder zur Messung einer Stoffkonzentration eines Analyten in einem Volumen oder einem Gewebe, insbesondere im Gewebe eines menschlichen oder tierischen Subjekts, wobei die Vorrichtung Folgendes umfasst:

eine Anregungsquelle, die zum Einstrahlen von Anregungsstrahlung bei einer oder mehreren Wellenlängen in das Volumen oder in das Gewebe eingerichtet ist, wobei die Anregungsstrahlung von dem darin enthaltenen Analyten absorbiert wird;

eine Detektionsvorrichtung mit einem Sensor zur direkten oder indirekten Erfassung von Reaktionen in dem Volumen oder Gewebe aufgrund der Absorption von Anregungsstrahlung, wobei die Reaktionen Antwortsignale, insbesondere in Form einer Temperaturerhöhung oder eine Druckwelle erzeugen, die einen Absorptionsgrad der Anregungsstrahlung repräsentieren oder aus denen ein Absorptionsgrad bestimmbar ist;

eine Steuerung zum Steuern der Anregungsquelle, wobei die Anregungsquelle mehrere Strahlungselemente, auch Anregungsquellenelemente genannt, aufweist, die jeweils dazu eingerichtet sind, Strahlung in einem bestimmten Wellenzahlbereich abzustrahlen, wobei die Wellenzahlbereiche verschiedener Strahlungselemente sich jeweils unterscheiden und wobei die Anregungsquelle wenigstens ein Strahlungselement enthält, das dazu eingerichtet ist in einem der folgenden Wellenzahlbereiche eine Anregungsstrahlung abzustrahlen, wobei die Breite der Wellenzahlbereiche kleiner als 15/cm, vorzugsweise kleiner als 10/cm, weiter vorzugsweise kleiner als 5/cm, weiter vorzugsweise kleiner als 3/cm oder kleiner als 2/cm ist:

Wellenzahlenbereiche die eine der folgenden Wellenzahlen, jeweils angegeben in 1/cm, enthalten: 1015, 1025, 1036, 1047, 1059, 1065, 1070, 1080, 1093, 1106, 1120, 1130, 1151, 1180, 1205 vorzugsweise Wellenzahlenbereiche, die eine der folgenden Wellenzahlen enthalten: 1015, 1025, 1036, 1047, 1059, 1070, 1080, 1093, 1106, 1120, 1151, 1180, 1205, weiter vorzugsweise eine der folgenden Wellenzahlen: 1015, 1036, 1059, 1070, 1080, 1093, 1106, 1120, 1151, 1180, 1205, weiter vorzugsweise eine der folgenden Wellenzahlen: 1015, 1036, 1059, 1120, 1180.

Bisher wurden zur Erfassung eines Analyten oder zur Messung einer Stoffkonzentration eines Analyten in einem Volumen oder einem Gewebe mittels ähnlicher Vorrichtungen vorwiegend als Anregungsquellen Laser eingesetzt, deren Wellenlängen/Wellenzahlen kontinuierlich veränderbar waren, um kontinuierliche Absorptionsspektren aufzunehmen, beispielsweise mit an sich bekannten Verfahren der Thermooptischen Spektroskopie.

Es sind auch bereits Laser-arrays vorgeschlagen worden, um Teilspektren auf bestimmte Wellenlängen gestützt aufzunehmen. Allerdings besteht dabei allgemein das Problem, die geeignetsten Wellenlängen oder Wellenzahlen für eine Messung auszuwählen, um mit einer möglichst geringen Zahl an Stützstellen eine möglichst gute Repräsentation eines Spektrums oder eine zuverlässige Identifizierung eines Stoffs zu erreichen. Dieses Problem ist in manchen Fällen noch gut handhabbar, falls ein Stoff unvermischt vorkommt und ein einziges Spektrum vorliegt. In dem praktischen Fall einer üblicherweise in biologischen Proben vorliegenden Mischung einer Vielzahl von Stoffen, bei denen weder bekannt ist, welche Stoffe vorkommen, noch, in welchem Mischungsverhältnis diese vorliegen, wird die Auswahl von geeigneten Wellenzahlen außerordentlich schwierig. Beispielsweise ist es in einem solchen Fall nicht mehr selbstverständlich, dass Absorptionsmaxima eines zu untersuchenden oder nachzuweisenden Stoffes eine gute Wahl sind, da gerade diese Maxima von hohen Absorptionsgraden anderer beigemischter Stoffe überlagert sein können. Die Auswahl von geeigneten Wellenzahlen muss somit berücksichtigen, welche anderen Stoffe beigemischt sein können und inwieweit die beigemischten Anteile dieser Stoffe variieren können. Es hat sich herausgestellt, dass die Auswahl aus den Wellenzahlenbereichen um die Wellenzahlen 1015, 1036, 1059, 1120 oder 1180 herum oder auch die Auswahl einiger oder aller genau dieser genannten Wellenzahlen gerade beim Nachweis der Glukosekonzentration in biologischem Gewebe, besonders in der interstitiellen Schicht der menschlichen Haut, gute Ergebnisse liefert (Wellenzahlen werden im vorliegenden Text stets in der Einheit 1/cm angegeben). Die weiteren angegebenen Wellenzahlenbereiche stellen sinnvolle Ergänzungen dar, wenn genügend Strahlungselemente verfügbar sind. Dies ist nicht immer unproblematisch, insbesondere, wenn die Anregungsstrahlung im mittleren Infrarotbereich liegen soll, wo vorteilhaft Quantenkaskadenlaser eingesetzt werden, die in der Form von Arrays mit beschränkten Zahlen von Strahlungselementen verfügbar gemacht worden sind.

Werden verschiedene Wellenzahlen zur Messung herangezogen, so können auch für verschiedene Wellenzahlen unterschiedliche Modulationscharakteristiken angewendet werden, die von der Dämpfung der Anregungsstrahlung bei der jeweiligen Wellenzahl abhängen können. Auf diese Möglichkeit wird weiter unten noch im Detail eingegangen.

Es kann weiter vorgesehen sein, dass die Anregungsquelle mehr als zwei, insbesondere mehr als 5, weiter insbesondere mehr als 8 oder mehr als 10 Strahlungselemente aufweist, wobei die Wellenzahlenbereiche in denen diese Strahlungselemente eingerichtet sind, Anregungsstrahlung abzustrahlen, sich unterscheiden, wobei die Breite der Wellenzahlenbereiche kleiner als 15/cm, vorzugsweise kleiner als 10/cm, weiter vorzugsweise kleiner als 5/cm, weiter vorzugsweise kleiner als 3/cm oder kleiner als 2/cm ist und wobei jedes der Strahlungselemente dazu eingerichtet ist, in einem der folgenden Wellenzahlenbereiche eine Anregungsstrahlung abzustrahlen:
Wellenzahlenbereiche, die eine der folgenden Wellenzahlen enthalten, jeweils angegeben in 1/cm: 1015, 1025, 1036, 1047, 1059, 1065, 1070, 1080, 1093, 1106, 1120, 1130, 1151, 1180, 1205, wobei vorzugsweise die Wellenzahlenbereiche eine der folgenden Wellenzahlen enthalten: 1015, 1025, 1036, 1047, 1059, 1070, 1080, 1093, 1106, 1120, 1151, 1180, 1205, weiter vorzugsweise eine der folgenden Wellenzahlen: 1015, 1036, 1059, 1070, 1080, 1093, 1106, 1120, 1151, 1180, 1205, weiter vorzugsweise eine der folgenden Wellenzahlen: 1015, 1036, 1059, 1120, 1180.

In Abhängigkeit von der biologischen Umgebung können aus den genannten Wellenzahlenbereichen die geeigneten Untergruppen ausgewählt werden. Dieser Vorgang der Auswahl kann auch bei einer Anwendung bei einem Menschen nach Kalibrierungsmessungen individuell getroffen werden. Zu diesem Zweck können dann mehrere Messungen mit unterschiedlichen Gruppen von Wellenzahlen oder einer Auswahl von Wellenzahlen aus unterschiedlichen Wellenzahlenbereichen durchgeführt, die Messergebnisse bewertet und danach kann eine Auswahl von Wellenzahlen aus den oben angegebenen Bereichen getroffen werden.

Weiter kann vorgesehen sein, dass die Steuerung eine Modulationseinrichtung zur zeitlichen Modulation der Anregungsstrahlung der Strahlungselemente, insbesondere jedes einzelnen der Strahlungselemente, aufweist.

Durch eine Modulation der Anregungsstrahlung können periodisch impulsartige Temperaturerhöhungen aufgrund der Absorption von Anregungsstrahlung in dem Volumen/Gewebe erzeugt werden, die mit verschiedenen Messmethoden auch quantitativ erfasst werden können. Beispiele für geeignete Messmethoden werden weiter unten beschrieben. Durch die Verwendung von Lock-in -Verstärkern bei der Signalverarbeitung können die auf den Nachweis der Absorption von Anregungsstrahlung zurückgehenden Signale gut isoliert werden. Als Modulationscharakteristiken kommen verschiedene Formen periodischer Funktionen, wie sinus-, rechteck- oder sägezahnförmige Funktionen in Frage, jedoch auch nicht periodische Impulsformen, deren Messergebnisse im Zeitbereich ausgewertet werden können.

In einer weiteren Implementierung kann vorgesehen sein, dass die Detektionseinrichtung einen Messkörper umfasst, der mit dem Volumen, insbesondere mit der Haut des Subjekts, in Kontakt gebracht werden kann, wobei die Übertragung von durch Absorption von Anregungsstrahlung in dem Volumen, insbesondere im Gewebe, erzeugten Wärme- und/oder Druckwellen auf einen mit dem Messkörper verbundenen oder in dem Messkörper gebildeten Sensor ermöglicht wird, wobei die Detektionsvor-richtung zum Erfassen einer physikalischen Reaktion des Sensors oder des Messkörpers oder einer darin enthaltenen Komponente auf eine Wärme- und/oder Druckwelle eingerichtet ist, die bei Absorption der Anregungsstrahlung durch den Analyten in dem Volumen oder dem Gewebe erzeugt und zum Messkörper übertragen wird, und zum Erzeugen eines Antwortsignals auf der Grundlage der erfassten physikalischen Reaktion, wobei das Antwortsignal einen Absorptionsgrad der Anregungsstrahlung repräsentiert oder,

dass die Detektionseinrichtung einen akustischen Sensor umfasst, der mit dem Volumen, insbesondere mit der Haut des Subjekts, in Kontakt gebracht werden kann, wobei die Übertragung von durch Absorption von Anregungsstrahlung in dem Volumen, insbesondere im Gewebe, erzeugten Druckwellen auf den akustischen Sensor ermöglicht wird, wobei die Detektionsvorrichtung zum Erfassen eines Signals des Sensors auf eine Druckwelle eingerichtet ist, die bei Absorption der Anregungsstrahlung durch den Analyten in dem Volumen oder dem Gewebe erzeugt und zum akustischen Sensor übertragen wird, und wobei die Detektionsvorrichtung zum Erzeugen eines Antwortsignals auf der Grundlage des erfassten Signals eingerichtet ist, wobei das Antwortsignal einen Absorptionsgrad der Anregungsstrahlung repräsentiert, oder

dass die Detektionseinrichtung einen optischen oder pyrometrischen Sensor umfasst, der auf das Volumen, insbesondere auf die Haut des Subjekts, gerichtet werden kann, und der Temperaturerhöhungen in dem Volumen oder Gewebe und/oder an der Oberfläche des Volumens oder Gewebes detektiert, wobei die Detektionsvorrichtung zum Erfassen eines Signals des optischen oder pyrometrischen Sensors eingerichtet ist, das bei Absorption der Anregungsstrahlung durch den Analyten in dem Volumen oder dem Gewebe durch eine Temperaturerhöhung erzeugt wird, und wobei die Detektionsvorrichtung zum Erzeugen eines Antwortsignals auf der Grundlage des erfassten Signals eingerichtet ist, wobei das Antwortsignal einen Absorptionsgrad der Anregungs-strahlung repräsentiert.

Das an erster Stelle aufgeführte Messverfahren ist in verschiedenen Ausprägungen bekannt. Es kann als Nachweis einer Druck- und/oder Wärmewelle beispielsweise in dem Messkörper eine Änderung des Brechungsindex detektiert werden, entweder durch Ablenkung eines Detektionslichtstrahls und Messung des Ablenkungsgrades, durch Messung einer erzeugten piezoelektrischen Spannung oder durch ein interferometrisches Verfahren. Einige der Verfahren kommen auch ohne einen Messkörper aus, der mit der Oberfläche des Volumens/Gewebes unmittelbar in Kontakt stehen muss.

In einer weiteren Realisierungsform kann vorgesehen sein, dass die Strahlungselemente jeweils dazu eingerichtet sind, Anregungsstrahlung mit einer einzigen, festen, jeweils in dem dem Strahlungselement zugeordneten Wellenzahlbereich liegenden, Wellenzahl abzustrahlen.

Es ist grundsätzlich auch denkbar, im Rahmen der beschriebenen Vorrichtung mehrere einstellbare Laser einzusetzen oder einen durchstimmbaren Laser so zu betreiben, dass er einzelne Wellenzahlen aus den angegebenen Wellenzahlenbereichen anfährt. Es ist jedoch in vielen Fällen einfacher, ein Laserarray mit festen Wellenzahlen einzusetzen. Durch bestimmte Effekte können dabei die einzelnen Wellenzahlen noch in gewissem beschränktem Umfang verändert werden, beispielsweise durch eine gezielte Temperatursteuerung. Auch dieser Effekt kann genutzt werden, um eine bestimmte Auswahl von Wellenzahlen zu erzeugen oder zu korrigieren. Dieses Vorgehen kann für eine individuelle Kalibrierung der Vorrichtung genutzt werden.

Es kann weiter vorgesehen sein, dass die Anregungsquelle ein Array mit mehreren, fest relativ zueinander angeordneten Strahlungselementen aufweist.

Durch eine solche Konstruktion werden nicht nur die passenden Strahlungselemente zur Verfügung gestellt, sondern ihre Energieversorgung und Temperaturregelung kann fest konfektioniert werden und auch eine geeignete optische Strahlführung der Anregungsstrahlen, die von verschiedenen Strahlungselementen stammen, kann mit in einen festen Aufbau integriert werden. Größere Teile und Elemente eines solchen Aufbaus können mit Epitaxieverfahren zuverlässig mit der erforderlichen Genauigkeit reproduzierbar hergestellt werden.

Wie bereits weiter oben angedeutet, kann die Vorrichtung in einigen Gestaltungen besonders vorteilhaft verwendet werden, wenn der Analyt Glukose ist, insbesondere wenn das Gewebe die Haut des Subjekts ist und der Analyt in einer interstitiellen Flüssigkeit der Haut vorhandene Glukose ist.

Es kann weiter vorgesehen sein, dass die Steuerung dazu eingerichtet ist, eine Konzentration des Analyten im Gewebe basierend auf dem von der Detektionsvorrichtung erzeugten Antwortsignal zu bestimmen.

Das Antwortsignal kann dabei je nach der eingesetzten Messmethode verschiedener Natur sein.

Es kann in einer weiteren Implementierung vorgesehen sein, dass eines, zwei, drei oder vier Strahlungselemente, die dazu eingerichtet sind, in den Wellenzahlbereichen abzustrahlen, die die Wellenzahlen 1015, 1036, 1059, 1120 oder 1180 enthalten, mit einer höheren Leistung betreiben werden, als alle übrigen Strahlungselemente, insbesondere mit mehr als 40 mW. Dabei kann auch vorgesehen sein, dass die betreffenden Strahlungselemente unmittelbar Anregungsstrahlung bei den angegebenen Wellenzahlen mit einer solchen Mindestleistung abstrahlen. Somit werden diese Wellenzahlen bei Auswertung des Absorptionsspektrums besonders stark gewichtet und/oder es wird auch einer bei diesen Wellenzahlen besonders starken Dämpfung der Anregungsstrahlung entgegengewirkt.

Es kann weiterhin vorgesehen sein, dass eines, zwei, drei oder vier oder mehr als 6 oder mehr als 9 oder alle Strahlungselemente, die dazu eingerichtet sind, in den Wellenzahlenbereichen abzustrahlen, die die Wellenzahlen 1015, 1036, 1059, 1070, 1080, 1093, 1106, 1120, 1151, 1180, 1205, enthalten, mit einer höheren Leistung betreiben werden, als alle übrigen Strahlungselemente, insbesondere mit mehr als 30 mW.

Auch die hier zusätzlich genannten Wellenzahlen können somit stärker gewichtet werden als andere Wellenzahlen, womit dem Umstand Rechnung getragen wird, dass beispielsweise bei den genannten Wellenzahlen ein besonders ungestörtes Signal vorliegt, weil dort Beimischungen von anderen Stoffen keine Absorption verursachen oder es kann auch hier einer erhöhten Dämpfung bei den angegebenen Wellenzahlen entgegengewirkt werden.

Eine weitere Ausgestaltung der Vorrichtung kann vorsehen, dass eines der Strahlungselemente dazu eingerichtet ist, Strahlung im Wellenzahlenbereich zwischen 900/cm und 930/cm, insbesondere bei einer einzigen, festen Wellenzahl, abzustrahlen und dass die Steuerung dazu eingerichtet ist, die Strahlungsleistung der Strahlungselemente auf der Grundlage des dieser Anregungsstrahlung im Wellenzahlenbereich zwischen 900/cm und 930/cm zugeordneten Antwortsignals zu steuern oder zu regeln.

Es hat sich gezeigt, dass die Absorption in dem genannten Wellenzahlenbereich weitestgehend unabhängig vom Glukosegehalt der Probe ist. Deshalb eignet sich eine Messung in diesem Bereich dazu, Informationen beispielsweise über die abgestrahlte Laserleitung und gegebenenfalls noch über andere, von der Glukosekonzentration unabhängige Parameter zu erhalten. Diese Information kann in der Steuerung zur Steuerung der Laserleistung und/oder zur Normierung der Messergebnisse dienen.

Die Erfindung bezieht sich außer auf eine Vorrichtung der oben beschriebenen Art auch auf ein Verfahren der oben beschriebenen Art zur Erfassung eines Analyten oder zur Messung einer Stoffkonzentration eines Analyten in einem Volumen, insbesondere im Gewebe eines menschlichen oder tierischen Subjekts, unter Verwendung einer Vorrichtung mit einer Anregungsquelle, die zum Einstrahlen von Anregungsstrahlung bei einer oder mehreren Wellenlängen in das Volumen oder in das Gewebe eingerichtet ist, wobei die Anregungsstrahlung von dem darin enthaltenen Analyten absorbiert wird,

mit einer Detektionsvorrichtung mit einem Sensor zur Erfassung von Temperaturerhöhungen in dem Volumen aufgrund der Absorption von Anregungsstrahlung, wobei die Temperaturerhöhung einen Absorptionsgrad der Anregungsstrahlung repräsentiert oder aus der Temperaturerhöhung ein Absorptionsgrad bestimmbar ist,

und mit einer Steuerung zum Steuern der Anregungsquelle, wobei die Anregungsquelle mehrere Strahlungselemente aufweist, die jeweils dazu eingerichtet sind, Strahlung in einem bestimmten Wellenzahlenbereich abzustrahlen, wobei die Wellenzahlenbereiche verschiedener Strahlungselemente sich jeweils unterscheiden und wobei die Anregungsquelle mehrere Strahlungselemente enthält, deren Wellenzahlenbereich jeweils eine der folgenden Wellenzahlen, jeweils angegeben in 1/cm, enthält: 1015, 1025, 1036, 1047, 1059, 1065, 1070, 1080, 1093, 1106, 1120, 1130, 1151, 1180, 1205,

wobei in einem Verfahrensabschnitt durch mehrere Strahlungsquellen je eine Test-Anregungsstrahlung erzeugt und jeweils die Test-Absorption der Test-Anregungsstrahlung in dem Volumen, insbesondere in dem Gewebe ermittelt wird und dass bei einer danach erfolgenden Erfassung des Analyten die Intensität und /oder eine Modulationsfrequenz der Anregungsstrahlung für die jeweiligen Strahlungsquellen in Abhängigkeit von der jeweils ermittelten Test-Absorption gesteuert wird.

Unter der Intensität der Anregungsstrahlung kann dabei auch die Laserleistung bei der jeweiligen Wellenzahl verstanden werden. Damit kann die Leistung der Anregungsstrahlung und die Modulationsfrequenz für jede Wellenzahl oder für verschiedene Wellenzahlenbereiche auf die wellenzahlabhängige Dämpfung bestimmt werden. Im Folgenden wird noch genauer erläutert, aus welchen Gründen die Modulationscharakteristik/Modulationsfrequenz für verschiedene Wellenzahlen oder Wellenzahlenbereiche unterschiedlich gewählt werden kann, um die Qualität der Messwerte zu erhöhen.

Im Folgenden wird die Erfindung anhand von Ausführungsbeispielen in Figuren einer Zeichnung gezeigt und nachfolgend erläutert.

Dabei zeigen

Fig. 1: Ein Messprinzip, das einigen Ausführungsformen der Erfindung zugrunde liegt;
Fig. 2: Das Absorptionsspektrum von Glucose in Wasser, abzüglich des Wasserhintergrunds;

Fig. 3: eine Schnittansicht einer Vorrichtung, die zur Durchführung von Ausführungsformen der Erfindung geeignet ist, die auf Antwortsignalen beruhen, die auf einer Ablenkung eines Detektionslichtstrahls basieren;

Fig. 4: Ergebnisse einer Clarke-Fehlergitteranalyse, die mit einem Gerät des in Fig.1 gezeigten Typs erzielt wurden.

Fig. 5: eine Vorrichtung, die zum Ausführen von Beispielen der Erfindung geeignet ist, die auf Reaktionssignalen basieren, die auf piezoelektrischen Reaktionen auf Wärme- oder Druckwellen basieren, die von dem der Analyse unterzogenen Gewebe empfangen werden;

Fig. 6: eine Vorrichtung, die zum Ausführen von Beispielen der Erfindung geeignet ist und auf Antwortsignalen beruht, die auf interferometrisch erfassten Phasenänderungen in einem Detektionslichtstrahl beruhen, der in einem Messkörper geführt wird;

Fig. 7: eine Vorrichtung zur Überwachung eines Analyten im Gewebe eines menschlichen oder tierischen Subjekts gemäß einer weiteren beispielhaften Ausführungsform der Erfindung;

Fig. 8: ein Hauptgehäuse einer Vorrichtung zur Überwachung eines Analyten im Gewebe eines menschlichen oder tierischen Subjekts gemäß einer beispielhaften Ausführungsform der Erfindung;

Fig. 9: eine Darstellung der Ausbreitung einer thermischen Welle im Material eines Probekörpers in Abhängigkeit von der Dämpfung der Anregungsstrahlung in dem Probekörper bei einem ersten Absorptionskoeffizienten;

Fig. 10: eine Darstellung der Ausbreitung einer thermischen Welle im Material eines Probekörpers in Abhängigkeit von der Dämpfung der Anregungsstrahlung in dem Probekörper bei einem zweiten Absorptionskoeffizienten, der größer ist als der erste Absorptionskoeffizient;

Fig. 11: eine Tabelle von Absorptionskoeffizienten, die als Beispiels für einen nachzuweisenden Stoff, beispielsweise Glukose, bei verschiedenen Wellenlängen der Anregungsstrahlung gelten und die verschiedenen Frequenzen der angewandten Modulationen;

Fig. 12: eine Auswahl von Wellenzahlen, jeweils angegeben in der Einheit 1/cm, die für eine nicht-invasive Glukosemessung sinnvoll sein können;

Fig. 13: eine Funktion, die für die jeweilige Tiefe im Messvolumen den bis zu dieser Tiefe bereits absorbierten Anteil der Anregungsstrahlung angibt und die somit auch die Dämpfungsfunktion repräsentiert; sowie

Figuren 14 und 15 eine Darstellung einer Anregungsquelle in zwei Ansichten von oben und von einer Seite.

Es versteht sich, dass sowohl die vorstehende allgemeine Beschreibung als auch die folgende Beschreibung lediglich beispielhaft und erläuternd sind und die hierin beschriebenen Methoden und Geräte nicht einschränken.

In dieser Anmeldung kann die Verwendung des Singulars auch den Plural umfassen, sofern nicht ausdrücklich etwas Anderes angegeben ist.

Außerdem bedeutet die Verwendung von "oder" gegebenenfalls "und/oder", sofern nicht anders angegeben.

In den Zeichnungen und der folgenden Beschreibung werden, soweit möglich, dieselben Bezugszeichen verwendet, um auf dieselben oder ähnliche Gegenstände zu verweisen.

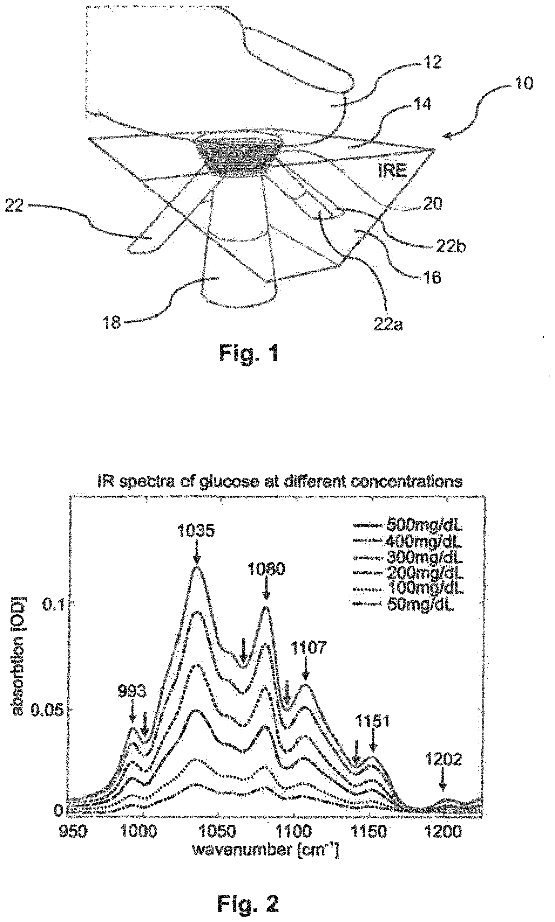

Fig. 1 ist eine schematische Darstellung eines möglichen Messprinzips, das dem im Folgenden näher beschriebenen Verfahren zur Analytmessung zugrunde liegt.

Während sich das Gerät und Verfahren der Erfindung für die Analyse verschiedener Proben oder Gewebearten sowohl von Menschen als auch von Tieren eignen, die mindestens einen Analyten enthalten, konzentriert sich die folgende Beschreibung auf spezielle Ausführungsformen, bei denen es sich bei der Probe oder dem Gewebe um die Haut eines Patienten und bei dem Analyten um Glukose in der interstitiellen Flüssigkeit der Haut handelt.

Es versteht sich, dass alle im Folgenden mit speziellem Bezug auf die Glukosemessung angegebenen Details und Erläuterungen auch für andere Gewebearten und Analyten gelten, sofern zutreffend, ohne dass sie im Folgenden explizit erwähnt werden.

In der Darstellung von Fig. 1 wird eine Fingerspitze 12 eines Benutzers in thermischen und/oder mechanischen Kontakt mit einer Kontaktfläche 14 eines Messkörpers 16 gebracht.

In einer alternativen, nicht dargestellten Ausführungsform kann die Fingerspitze über eine Akustikzelle akustisch mit dem Messkörper gekoppelt sein. Die Akustikzelle kann einen mit Flüssigkeit oder Gas gefüllten Hohlraum umfassen, der die Übertragung von Druckwellen auf den Messkörper ermöglicht und es kann in der Akustikzelle ein Mikrofon zur Aufnahme und Messung von akustischen wellen vorgesehen sein.

Gemäß der in Fig. 1 dargestellten Konstruktion wird ein Anregungsstrahl 18 durch Luft oder durch einen Wellenleiter (nicht gezeigt) auf den Messkörper 16 und dann durch den Messkörper 16 hindurch und in die Haut an der Fingerspitze 12 geführt.

Zur Ermittlung der Konzentration der Glukose in der Haut, insbesondere in der interstitiellen Flüssigkeit der Haut, werden zur Absorptionsmessung nacheinander oder zumindest teilweise gleichzeitig verschiedene Wellenlängen der Anregungsstrahlung 18 gewählt, so dass aus den gemessenen Absorptionswerten ein Absorptionsspektrum gewonnen und die Konzentration der Glukose bestimmt werden kann.

In Fig. 2 sind Absorptionsspektren für verschiedene Konzentrationen von Glucose in Wasser dargestellt, wobei der Beitrag der Absorption durch Wasser abgezogen wurde.

Wie man dort sehen kann, weist das Glucosemolekül mehrere charakteristische Absorptionsspitzen im mittleren Infrarotbereich bei Wellenzahlen zwischen 993/cm und 1202/cm auf, was jeweils Wellenlängen im Bereich von 10,07 μm bis 8,32 μm entspricht.

Zwischen benachbarten Absorptionsspitzen sind lokale Absorptionsminima zu erkennen, die in der Abbildung durch vertikale Pfeile ohne Wellenzahlen gekennzeichnet sind.

Wie aus Fig. 2 ersichtlich, können insbesondere die Unterschiede in der Absorption an den Absorptionspeaks und den lokalen Absorptionsminima charakteristisch für die Glucosekonzentration sein. Welche Wellenlängen für die Messung günstig sind, hängt jedoch auch von den Störgrößen ab, beispielsweise von der Absorption bei den betreffenden Wellenlängen durch andere Materialien, deren Konzentration nicht bestimmt werden soll.

Je größer die bei einer Wellenlänge die Absorption durch Glukose und je geringer bei derselben Wellenlänge die Absorption durch störende andere in der Probe vorhandene Stoffe ist, umso vorteilhafter und aussagekräftiger kann eine Messung der Absorption bei dieser Wellenlänge für die Bestimmung der Glukosekonzentration sein.

Um die Glukosekonzentration bestimmen zu können, kann es dementsprechend vorteilhaft sein, die Absorption an einigen oder allen Absorptionsspitzen oder in deren Nähe und an einigen oder allen lokalen Absorptionsminima und möglicherweise auch an einigen Punkten zwischen den Maxima und Minima zu messen.

Diese Wellenlängen werden hier als "für den Analyten (Glucose) charakteristische Wellenlängen" bezeichnet.

Es ist jedoch zu beachten, dass die Absorptionsfähigkeit beim niedrigsten lokalen Minimum bei 1140 cm-1 immer noch 18 % der Absorptionsfähigkeit beim höchsten Peak bei 1035 cm-i im relevanten Teil des Spektrums beträgt.

Dementsprechend hängt die Absorption bei jeder dieser Wellenlängen deutlich von der Konzentration der Glukose ab, sodass diese Wellenlängen für die Glukose charakteristisch sind, also "glukosecharakteristische Wellenlängen" genannt werden können.

Im Gegensatz dazu ist bei etwa 1180 cm-1 der Absorptionsgrad praktisch Null und damit ein globales und kein lokales Minimum, und diese Wellenlänge ist offensichtlich nicht charakteristisch für die Glukose (kann aber beispielsweise für Referenzmessungen verwendet werden, um etwa einen Kontaktdruck zwischen dem Messkörper 16 und der Haut der Fingerkuppe 12 zu bestimmen).

Unter "analytcharakteristischen Wellenlängen" können das gesamte Spektrum, jedoch in einer speziellen Ausprägung auch solche Wellenlängen verstanden werden, bei denen der Absorptionsunterschied zu dem nächstgelegenen Absorptionspeak oder nächstgelegenen lokalen Absorptionsminimum weniger als 30 %, vorzugsweise weniger als 20 %, des Absorptionsunterschieds zwischen dem nächstgelegenen Absorptionspeak und dem nächstgelegenen lokalen Absorptionsminimum beträgt.

Die Intensität des Anregungsstrahls 18 wird zeitlich periodisch mit einer bestimmten Frequenz f, insbesondere mit einer Sinus-, Rechteck oder Sägezahnform, moduliert, sodass die Anregungsstrahlung, in diesem Fall das Anregungslicht, abwechselnd Intervalle hoher Intensität und niedriger oder sogar verschwindender Intensität aufweist.

Ohne die Modulation auf eine bestimmte Wellenform beschränken zu wollen, werden Intervalle hoher Intensität im Folgenden als "Anregungslichtimpulse" bezeichnet.

Während der Anregungslichtimpulse wird Anregungslicht mit der für Glucose charakteristischen Wellenlänge in dem Gewebe absorbiert, so dass dort die Strahlungsenergie in Wärme umgewandelt wird.

Da sich die Glucosemoleküle innerhalb einer extrem kurzen Zeit aus dem angeregten Zustand unter Aussendung einer Wärmewelle entspannen, kann die Erzeugung eines entsprechenden Wärmeimpulses und/oder einer Druckwelle aus praktischen Gründen als augenblicklich und vor dem nächsten Anregungsstrahlungsimpuls erfolgend angesehen werden.

Somit entstehen neben den anregenden Anregungsstrahlungs- oder Lichtpulsen auch lokale Wärmepulse am Absorptionsort, die zu einem räumlich und zeitlich variierenden Temperaturfeld führen, welches man als thermische Welle bezeichnen kann.

Wie oben erläutert, ist der Begriff "Wärmewelle" etwas irreführend, da die Wärmebewegung durch das Material nicht durch eine Wellengleichung, sondern durch eine Diffusionsgleichung bestimmt wird.

Allerdings ist der Begriff "Wärmewelle" zumindest insoweit richtig, als dass sich bei einer Messung an der Haut eines Menschen Wärmeimpulse oder mit diesen korrespondierende Druckwellen vom Inneren der Haut zur Oberfläche 14 des Messkörpers 16 und in den Messkörper 16 hinein ausbreiten, wie man es von einer Wellenausbreitung gewohnt ist.

Ein thermischer Gradient 20, der durch einen solchen Wärmeimpuls verursacht wird, ist schematisch in Fig.1 im Inneren des Messkörpers dargestellt. Die Temperaturerhöhung, die auch mit einer Druckwelle einhergeht und die Dielektrizitätskonstante in dem Material des Messkörpers beeinflusst, geht von der Kontaktfläche des Messkörpers 16 mit der Haut aus und erstreckt sich kissenförmig in den Messkörper hinein.

Die vom Messkörper 16 von der Haut der Fingerspitze 12 aufgenommene Wärme verursacht eine physikalische Reaktion, die mit einem von verschiedenen möglichen Detektionsgeräten detektiert werden kann, die dazu ausgelegt sind, auf der Grundlage der physikalischen Reaktion ein Antwortsignal zu erzeugen, wobei dieses Antwortsignal den Absorptionsgrad des Anregungslichts angibt.

Nachfolgend werden verschiedene Möglichkeiten zur Erfassung der physikalischen Reaktion und zur Generierung geeigneter Antwortsignale beschrieben.

Unabhängig von der genauen Art der Erfassung der physikalischen Reaktion ist jedoch anzumerken, dass die maximale Tiefe unter der Hautoberfläche, in der die Absorption mittels zum Messkörper 16 wandernder Wärmeimpulse erfasst werden kann, in guter Näherung durch die thermische Diffusionslänge $\mu$ der Haut begrenzt ist, die als

$$\mu = \sqrt{\frac{\alpha}{\pi f}}$$

definiert ist, wobei die thermische Diffusivität $\alpha$ nach der Gleichung

$$\alpha = k\rho c$$

von der Massendichte $\rho$, der spezifischen Wärmekapazität c und der Wärmeleitfähigkeit k des Gewebes sowie von der Modulationsfrequenz f des Anregungslichts abhängt.

Demgemäß lässt sich durch die Wahl der Modulationsfrequenz f eine Tiefe definieren, die mit der thermischen Diffusionslänge korrespondiert und bis zu der sich eine etwaige Absorption des Anregungslichts in den am Messkörper 16 empfangenen Wärmepulsen niederschlägt.

Unter erneuter Bezugnahme auf Fig. 1 ist in der gezeigten Ausführungsform die physikalische Reaktion auf die von der Haut aufgenommene Absorptionswärme durch eine Änderung des Brechungsindex in einem Bereich nahe der Oberfläche 14 des Messkörpers 16 gebildet wo der Bereich mit einem hohen Wärmegradient 20 dargestellt ist.

Diese lokale Änderung des Brechungsindex bildet für einen eingestrahlten Detektions- Lichtstrahl 22 eine Art thermische Linse, die durch eine variierende Ablenkung des Detektionslichtstrahls detektiert werden kann.

Der Detektions-Lichtstrahl 22 durchläuft die thermische Linse bzw. den Wärmegradientenbereich und wird dann an der Berührungsfläche des Messkörpers 16 und der Haut der Fingers 12 reflektiert.

Bei jedem der periodisch aufeinander folgenden Wärmeimpulse auf der Haut kommt es zu einer lokalen Brechungsindexänderung durch Wechselwirkung mit dem Material des Messkörpers, die im Bereich der thermischen Linse zu einer Ablenkung des Detektionsstrahls 22 führt.

In Fig. 1 entspricht das Bezugszeichen 22b dem nicht abgelenkten Detektionsstrahl 22, während das Bezugszeichen 22a dem Detektionsstrahl entspricht, wenn er aufgrund der im Wärmegradientenbereich 20 gebildeten thermischen Linse abgelenkt wird.

Diese Auslenkung ist messbar und stellt ein Beispiel für das oben genannte Antwortsignal dar.

Der Grad der Ablenkung ist durch eine positionssensitive Fotodiode nachweisbar und bildet einen quantitativen Hinweis auf die empfangene Wärmemenge und damit auf den Grad der Absorption des Anregungslichts 18 in der Haut des Fingers 12.

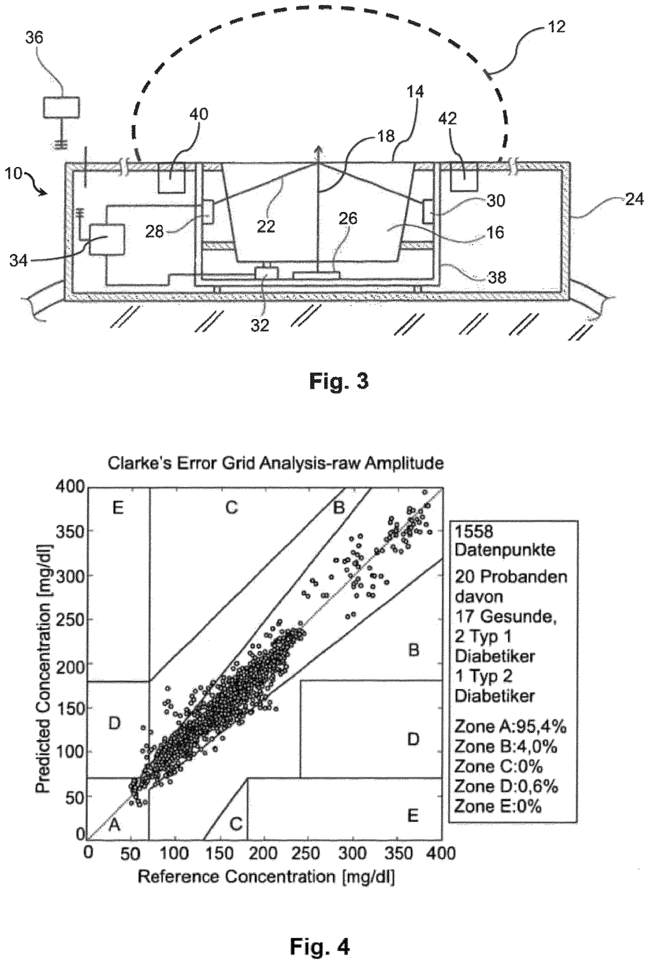

Fig. 3 zeigt eine detailliertere Schnittansicht einer Vorrichtung 10, die auf dem Messprinzip basiert, wie es unter Bezugnahme auf Fig 1. Beschrieben ist.

Das Gerät 10 umfasst ein Gehäuse 24, das den Messkörper 16 enthält und eine obere Oberfläche (Kontaktfläche) 14 aufweist, auf der ein Körperteil des Probanden/Patienten, beispielsweise ein Finger 12, ruht.

Innerhalb des Gehäuses 24 ist eine Anregungslichtquelle (auch Anregungsstrahlungsquelle oder Anregungsquelle genannt) 26 vorgesehen, die den Anregungslichtstrahl 18 erzeugt.

In der gezeigten Ausführungsform umfasst die Anregungsquelle 26 eine Anordnung von mehreren Quantenkaskadenlasern (nicht gezeigt), beispielsweise in der Form eines Laserarrays, von denen jeder eine eigene Wellenlänge aufweist.

Beispielsweise könnte die Anordnung aus Quantenkaskadenlasern einzelne Quantenkaskadenlaserelemente mit Wellenlängen umfassen, von denen wenigstens einige den in Fig. 2 gezeigten Absorptionsspitzen und lokalen Minima (d. h. den für Glukose charakteristischen Wellenlängen) entsprechen, sowie anderen Wellenlängen, die für Referenzmessungen verwendet werden können (z. B. zum Bestimmen eines Kontaktdrucks zwischen dem Messkörper 16 und der Haut der Fingerspitze 12, beispielsweise eine oder mehrere charakteristische Wellenlängen von Wasser) oder zum Erkennen anderer Substanzen, die die Glukosemessung stören könnten, beispielsweise Laktat oder Albumin. Grundsätzlich können die Wellenlängen oder Wellenlängenbereiche der einzelnen Lichtquellen der Anregungsquelle einer günstigen Auswahl für eine selektive Messung des zu erfassenden Stoffs in der vorliegenden Umgebung mit den gegebenen Störgrö-$\beta$en/Beimengungen anderer Stoffe entsprechen.

Die Vorrichtung 10 umfasst die Lichtquelle 28, beispielsweise einen Laser zum Aussenden des Detektionsstrahls 22, sowie einen positionsempfindlichen Detektor oder Sensor 30 (z. B. eine Quadrantenfotodiode), der eine Erfassung der Ablenkung des Detektionsstrahls 22 ermöglicht.

Die Lichtquelle 28 und der positionsempfindliche Detektor 30 bilden zusammen ein Beispiel für eine Erfassungsvorrichtung zum Erfassen der physikalischen Reaktion des Messkörpers 16 auf von der Probe/der Fingerspitze 12 empfangene Wärme- und/oder Druckwellen.

Der Messkörper 16 ist dabei sowohl für den Anregungslichtstrahl 18 als auch für den Detektionslichtstrahl 22 transparent.

Darüber hinaus ist in einem Beispiel eine Kamera 32 oder ein anderes bildgebendes Gerät vorgesehen, das es ermöglicht, Bilder der Kontaktfläche 14 des optischen Mediums 16 aufzunehmen, um dadurch ein Hautmuster, beispielsweise einen Fingerabdruck des auf der Kontaktfläche 14 aufliegenden Fingers 12, aufzuzeichnen.

Dieses Hautmuster kann von einer Steuereinheit 34 verarbeitet werden, um beispielsweise einen Benutzer anhand seines Hautmusters (z. B. Fingerabdruck) zu identifizieren und/oder zu authentifizieren.

Die Steuereinheit 34 dient außerdem zur Steuerung der Lichtquellen 26 und 28 für das Anregungslicht bzw. das Detektionslicht sowie des Sensors 30.

Die Steuereinheit 34 bildet ein Beispiel für einen "Controller".

Die Steuereinheit 34 kann auch drahtlos mit einem externen Datenverarbeitungsgerät 36 in Verbindung stehen, um Daten auszutauschen.

Über die drahtlose Verbindung können beispielsweise benutzerspezifische Kalibrierungsdaten für den über den Fingerabdruck identifizierten Benutzer von der Steuereinheit 34 abgerufen werden.

In einigen Beispielen können die Steuereinheit 34 und das externe Datenverarbeitungsgerät 36 zusammen ein Beispiel eines "Controllers" bilden.

Der Controller kann einen oder mehrere Prozessoren, Mikrocontroller, Computer, ASICs, FPGAs oder dergleichen umfassen.

Die Steuerung kann verteilt sein, wie in Fig. 3 dargestellt, mit verschiedenen Komponenten, die miteinander in Datenkommunikation stehen, oder sie kann aus einer einzigen Steuereinheit, beispielsweise der Steuereinheit 34, bestehen, die für alle Steuerfunktionen ausgelegt ist.

Der Controller kann allgemein in Hardware, in Software oder einer Kombination aus beidem verkörpert sein.

Wie in Fig. 3 weiter zu sehen ist, sind die Anregungs- und Detektionslichtquellen 26 und 28 sowie der positionsempfindliche Detektor 30 alle an einer gemeinsamen Trägerstruktur 38 befestigt.

Dies bedeutet, dass diese Komponenten präzise auf dieser Struktur 38 vormontiert werden können, sodass sie beim Zusammenbau der Vorrichtung 10 nicht einzeln eingestellt oder kalibriert werden müssen. Es kann an dem Träger auch eine Justiereinrichtung zur relativen Positionierung der genannten Komponenten vorgesehen sein.

Darüber hinaus umfasst das Gerät 10 eine Corneometrie-Einrichtung 40, die eine Messung des Wassergehalts der Haut ermöglicht.

Corneometrische Geräte zur Messung des Wassergehalts in der oberen Hautschicht sind an sich in der Technik bekannt und müssen hier nicht im Detail beschrieben werden.

Beispielsweise messen bekannte corneometrische Geräte die Impedanz, insbesondere kapazitive Impedanz, der Haut mithilfe zweier Interdigitalelektroden, an die eine Wechselspannung angelegt wird.

Das corneometrische Gerät 40 aus Fig. 3 steht in Kontakt mit der Fingerspitze 12, wenn diese auf der Kontaktfläche 14 des Messkörpers 16 aufliegt.

Das corneometrische Gerät 40 ist ein Beispiel für die oben genannten "Hilfssensoren", d. h. ein Sensor, der an sich nichts mit der Messvorrichtung zur Messung der Analytabsorption zu tun hat, wobei die Messdaten des Hilfssensors jedoch zur Kalibrierung und/oder zur Interpretation der Analytabsorptionsmessung herangezogen werden können.

Das Gerät umfasst außerdem als beispielhaften Hilfssensor einen pH-Sensor 42 zur Messung des pH-Wertes der Haut. pH-Sensoren zur Messung des pH-Wertes auf Oberflächen, auch auf der Haut, sind an sich aus dem Stand der Technik bekannt und müssen hier nicht näher beschrieben werden. pH-Sensoren zur Messung des pH-Wertes der Haut sind für medizinische, aber auch kosmetische Zwecke kommerziell erhältlich.

Fig. 4 zeigt Ergebnisse einer Clarke's Error Grid-Analyse, die bei einer Messung mit einem Gerät des in Fig. 3 gezeigten Typs erzielt wurden, und verdeutlicht, dass mit dem anhand der Fig. 1 bis 3 beschriebenen Messverfahren tatsächlich sehr zuverlässig Blutzuckerkonzentrationen auf rein nicht-invasive Weise gemessen werden können.

Die in Fig. 4 dargestellten Daten sind der WO 2017/09782 A1 entnommen.

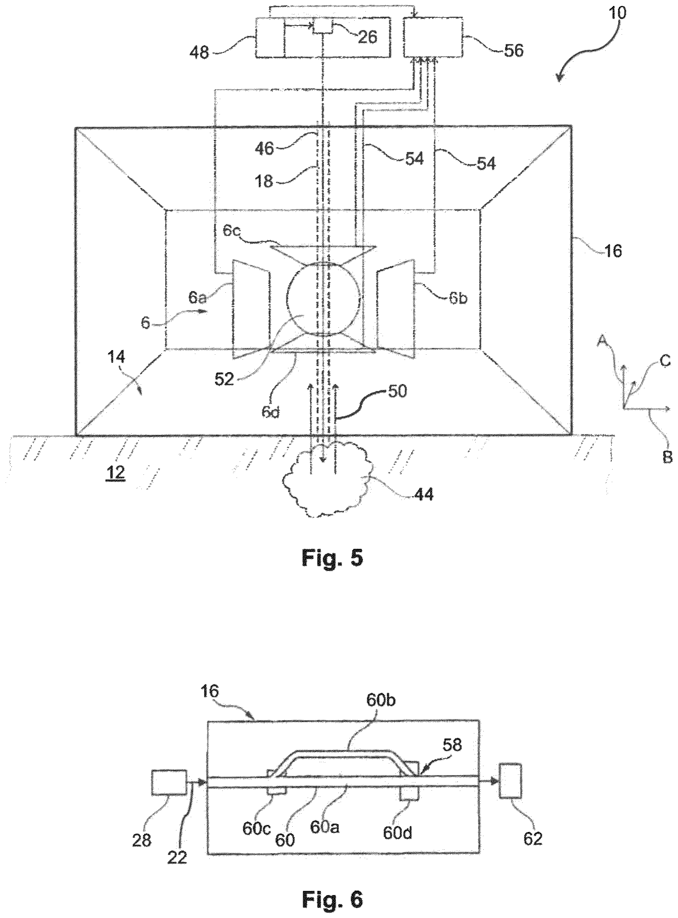

Fig. 5 zeigt schematisch eine Vorrichtung 10, die auf dem gleichen allgemeinen Prinzip wie die von Fig. 1 und 3 beruht, nämlich der Absorption von Anregungsstrahlung zur Erzeugung von Wärmeimpulsen, die vom Messkörper 16 aus der Probe/aus dem Gewebe der Fingerspitze/des Körperteils 12 empfangen werden, wobei sich aber die Messung von dem oben beschriebenen Verfahren in der genutzten physikalischen Reaktion und in der Art und Weise unterscheidet, wie die entsprechenden Antwortsignale erzeugt werden.

Eine solche Vorrichtung 10 sowie eine große Anzahl von Variationen davon sind im Detail in WO 2019/11059782 beschrieben, auf die hier Bezug genommen wird, so dass auf eine detaillierte Beschreibung hier verzichtet werden kann.

Wie zuvor umfasst die Vorrichtung einen Messkörper 16 mit einer Oberfläche 14, die mit der Probe in Form der Haut eines Fingers 12 in Kontakt gebracht oder gekoppelt wird.

Außerdem ist eine Anregungsquelle 26 für einen Anregungslichtstrahl 18 mit modulierter Intensität vorgesehen, der in einen Bereich 44 unterhalb der Oberfläche der Haut 12 eingestrahlt und dort absorbiert wird.

Der Anregungslichtstrahl 18 verläuft bei dieser Ausführungsform durch eine gestrichelt angedeutete Bohrung 46 durch den Messkörper 16, so dass der Messkörper 16 in diesem Fall selbst hierfür nicht transparent sein muss.

Zur Modulation der Intensität des Anregungslichtstrahls 18 ist eine Steuereinheit 48 vorgesehen.

Die Modulation kann, wie auch im Falle der in den Figuren 1 und 3 gezeigten Anordnung und allen anderen in diesem Text erläuterten Messeinrichtungen, im Allgemeinen auf verschiedene Arten erfolgen, einschließlich eines mechanischen Zerhackers/"choppers" oder eines Elements mit einem Transmissions- oder Reflexionsvermögen, das elektronisch gesteuert werden kann.

In bevorzugten Ausführungsformen wird die Intensität jedoch durch Modulieren der Ein-/Ausschaltzeiten der Anregungslichtquelle 26 sowie ihrer Leistung oder des Betriebsstroms während der Einschaltzeiten derselben moduliert.

Die Steuereinheit 48 kann beispielsweise zumindest teilweise in eine Steuerung wie die Steuereinheit 34 aus Fig. 1 integriert sein.

Eine durch die zeitlich variierende Absorption des intensitätsmodulierten Anregungsstrahls 18 im Bereich 44 der Haut 12 verursachte Wärmewelle, die durch Pfeile 50 symbolisiert wird, tritt in den Messkörper 16 ein und kann dort in einem Detektionsbereich 52, der piezoelektrische Eigenschaften aufweist, detektiert werden.

Die durch die aufgenommene Wärme 50 bzw. durch Druckwellen entstehenden Druckänderungen führen in dem Detektionsbereich 52 zu elektrischen Signalen, die mit den Elektroden 6a bis 6d erfasst werden können, welche über Leitungen 54 mit einem Auswertegerät 56 zur Analyse des Gewebes (in diesem Fall der Haut einer Fingerspitze) verbunden sind.

Die Elektroden 6a bis 6d können in einigen Beispielen zusammen mit der Auswertevorrichtung 56 ein Beispiel für eine Detektionsvorrichtung im Sinne der Erfindung bilden.

Bei der Auswerteeinrichtung 56 kann es sich um eine digitale Verarbeitungseinrichtung, beispielsweise einen Mikrocontroller oder Prozessor oder einen Computer handeln.

In einigen Beispielen kann die Auswertevorrichtung zumindest teilweise in eine Steuerung, wie etwa die Steuereinheit 34 aus Fig. 1, integriert sein.

In diesem Beispiel ähnelt die Druckänderung der physikalischen Reaktion des Messkörpers 16 oder einer anderen darin enthaltenen Komponente auf Wärme, die vom Gewebe der Fingerspitze 12 bei Absorption der Anregungsstrahlung aufgenommen wird, was mithilfe der piezoelektrischen Eigenschaften des Messkörpers 16 in der Form von elektrischen Signalen gemessen wird. Die Elektroden 6a bis 6d, ermöglichen die Messung dieser Signale, die das Antwortsignal darstellen, das den Grad der Absorption der Anregungsstrahlung 18 angibt.

In alternativen Varianten, die beispielsweise in der internationalen Anmeldung PCT/EP2019/064356 offenbart sind, auf die hier Bezug genommen wird, kann die Detektionsvorrichtung eine interferometrische Vorrichtung umfassen, die die Beurteilung der Phasenänderung eines ersten Teils des Detektionsstrahls relativ zu einem zweiten Teil des Detektionsstrahls ermöglicht, wobei nur einer der Teile des Detektionsstrahls, der einen Messarm durchläuft, durch die Auswirkungen der Wärme- oder Druckwelle im Messkörper 16 beeinflusst wird, und ein Antwortsignal erzeugt, die Phasenänderung und damit das Maß der Änderung eines Brechungsindex anzeigt.

In diesem Beispiel ist die physikalische Reaktion des Messkörpers 16 (oder einer darin enthaltenen Komponente) auf die von dem Material 12 aufgenommene Wärme bei Absorption der Anregungsstrahlung 18 wiederum eine lokale Änderung des Brechungsindex, während das Antwortsignal in diesem Fall ein interferometrisches Signal ist, das eine Änderung der Phase eines Teils des Detektionsstrahls aufgrund der lokalen Änderung des Brechungsindex widerspiegelt.

Dies ist schematisch in Fig. 6 dargestellt, wo ein Messkörper 16 gezeigt ist, der mit der Haut eines Körperteils (z. B. des Fingers, in dieser Fig. nicht dargestellt) in Kontakt gebracht wird.

In diesem Beispiel kann der Messkörper 16 ein Siliziumsubstrat sein, in dem eine Lichtleitstruktur 58 vorgesehen ist, die eine interferometrische Vorrichtung 60, beispielsweise in Form eines üblichen Interferometers oder eines Loop- Interferometers bildet.

Das interferometrische Gerät 60 bildet im dargestellten Fall ein Mach-Zehnder-Interferometer mit einem Messarm 60a und einem Referenzarm 60b.

Von einer Detektionslichtquelle 28 erzeugtes Detektionslicht 22 wird in die Lichtleitstruktur 58 eingespeist und von einem Splitter 60c in einen Abschnitt oder Teil des Detektionsstrahls, der entlang des Messarms 60a verläuft, und einen Abschnitt oder Teil, der entlang des Referenzarms 60b verläuft, aufgeteilt, wobei die Abschnitte dann von einem Kombinierer 6od zusammengeführt werden.

Der Messkörper 16 wird so verwendet bzw. angeordnet, dass der Referenzarm 60a der von der Haut bei Absorption von Anregungslicht ausgesandten Wärme- oder Druckwelle ausgesetzt ist, der Referenzarm 60b jedoch nicht oder zumindest in deutlich geringerem Maße.

Aufgrund der aufgenommenen Wärme ändert sich der Brechungsindex im Messarm 60a, was zu einer Phasenverschiebung des entlang des Messarms 60a laufenden Detektionslichts 22 führt.

Da das entlang des Referenzarms 60b wandernde Licht von der aufgenommenen Wärme unbeeinflusst bleibt, kommt es zu einer Änderung der relativen Phase der beiden durch den Kombinierer 60d kombinierten Lichtanteile, was zu einem Interferenzmuster führt, das mit einem Detektor 62 erkannt werden kann.

Dementsprechend können die Lichtquelle 28, die interferometrische Vorrichtung 60 und der Detektor 62 zusammen ein Beispiel für eine Detektionsvorrichtung bilden.

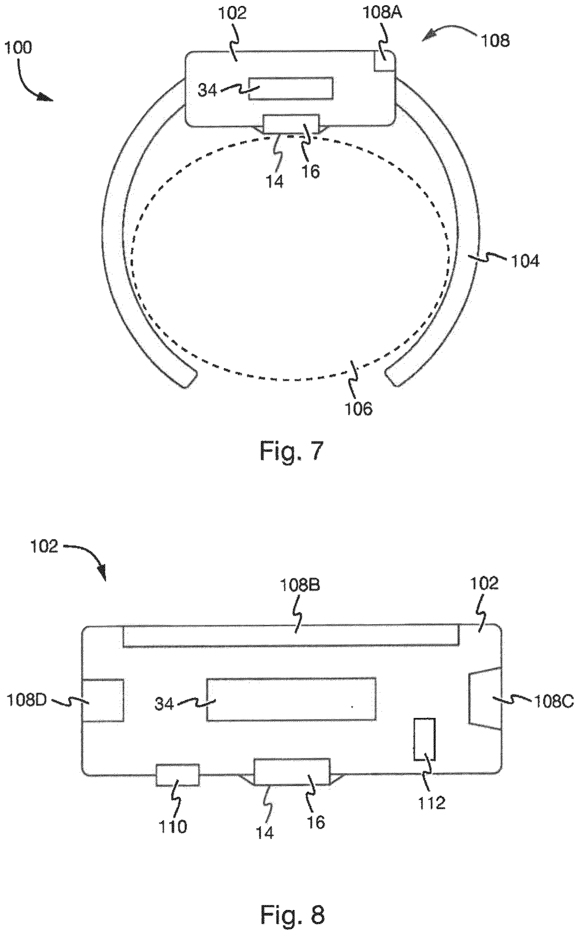

Fig. 7 zeigt eine schematische Darstellung einer Vorrichtung 100 zum Überwachen eines Analyten im Hautgewebe eines menschlichen oder tierischen Subjekts gemäß einer beispielhaften Ausführungsform der Erfindung.

Das Gerät 100 umfasst ein Hauptgehäuse 102 und einen Halteabschnitt 104 zum Befestigen des Geräts 100 an einem Körperteil 106 eines menschlichen oder tierischen Subjekts, beispielsweise an einer Extremität wie an einem Oberarm oder einem Handgelenk eines Benutzers/Patienten.

Der Halteabschnitt 104 ist dazu ausgebildet, um den Körperteil 106 herum angeordnet zu werden, so dass er sich zumindest teilweise, wie in Fig. 7 dargestellt, oder vollständig um den Körperteil 106 herum erstreckt.

Der Halteabschnitt 104 kann beispielsweise ein Armband, ein Gurt oder eine ähnliche Struktur sein.

Das Gerät 100 kann außerdem ein Display (nicht dargestellt) umfassen, das auf oder in einer oberen Oberfläche oder Wand des Hauptgehäuses angeordnet sein kann, beispielsweise ähnlich dem Display 108B in Fig. 8.

In einigen Beispielen kann das Gerät 100 ein tragbares Gerät, insbesondere eine Smartwatch oder ein Fingerring, sein. Es kann als Energiequelle eine aufladbare elektrische Batterie enthalten.

Im Folgenden wird bei dem Gerät die Richtung, die sich azimutal um das Körperteil 106 erstreckt, als Umfangsrichtung bezeichnet und eine Richtung, die senkrecht zur Oberfläche (d. h. der Haut) des Körperteils 106 verläuft, wird als radiale Richtung bezeichnet.

Das Gerät umfasst eine Vorrichtung zum Nachweis eines Analyten (z. B. Glukose) im Gewebe des Körperteils 106, z. B. in der Haut, beispielsweise unter Verwendung einer der weiter oben beschriebenen Nachweis- oder Messmethoden.

Die Vorrichtung zum Nachweis des Analyten ist im Hauptgehäuse 102 angeordnet und kann beispielsweise einer Vorrichtung aus einer der Fig. 3, 5 oder 6 ähnlich sein.

Insbesondere umfasst die Vorrichtung einen Messkörper 16 mit einer Kontaktoberfläche 14, die geeignet ist, mit der Haut des Subjekts, das die Vorrichtung 100 trägt, in Kontakt gebracht zu werden, um die Übertragung von Wärme- und/oder Druckwellen von dem Körperteil 106 auf den Messkörper 16 zu ermöglichen, die durch Absorption von Anregungsstrahlung in dem Gewebe erzeugt werden.

Die Vorrichtung umfasst weiterhin eine Anregungsquelle (nicht gezeigt) zum Einstrahlen von Anregungsstrahlung mit mehreren für den Analyten geeigneten oder charakteristischen Wellenlängen in das Gewebe und eine Detektionsvorrichtung (nicht gezeigt) zum Detektieren einer physikalischen Reaktion des Messkörpers 16 auf Wärme und/oder Druckwellen, die vom Gewebe bei Absorption der Anregungsstrahlung empfangen werden, und zum Erzeugen eines Antwortsignals basierend auf der erfassten physikalischen Reaktion.

Das Gerät 100 umfasst außerdem eine Steuerung 34 zur Steuerung der Anregungsquelle und der Detektionsvorrichtung zur Durchführung von Analytmessungen.

Der Messkörper, die Anregungsstrahlungsquelle, die Detektionseinrichtung und die Steuerung 34 können beispielsweise wie oben anhand der Figuren beschrieben ausgeführt sein. 3,5 und 6. In einem Beispiel ähnelt oder entspricht das Hauptgehäuse 102 dem Gehäuse 24 des Geräts 10 aus Fig. 3.

In einigen Beispielen kann der Controller 34 zumindest teilweise in eine Hauptsteuereinheit des Geräts 100 integriert sein, wobei die Hauptsteuereinheit beispielsweise die Anzeige des Geräts 100 steuern und so konfiguriert sein kann, dass sie Smartwatch-Funktionen in bekannter Art und Umfang bereitstellt.

Im Beispiel von Fig. 7 ist der Messkörper 16 in einer Bodenfläche oder Bodenwand des Hauptgehäuses 102 (z. B. gegenüber dem Display des Geräts 100) angeordnet, sodass der Messkörper 106 aus der Bodenfläche oder Wand hervorragt.

Wenn das Gerät 100 am Körperteil 106 befestigt ist, ist die Kontaktfläche 14 dem Körperteil 106 zugewandt.

Eine Verbindungsstelle zwischen der Bodenfläche oder Wand des Geräts 100 und dem Messkörper 106 kann durch eine Gummidichtung abgedeckt oder abgedichtet sein, die beispielsweise auch freiliegende Teile der Seitenwände des Messkörpers 106 abdecken oder abdichten kann.

In einigen Beispielen kann das Gerät 100 außerdem Betätigungsmittel (nicht gezeigt) zum vorübergehenden Erhöhen eines Kontaktdrucks zwischen dem Messkörper 16 und der Haut des Subjekts (d. h. der Haut des Körperteils 106) umfassen.

Die Betätigungsmittel können beispielsweise einen Aktuator (nicht dargestellt) umfassen, beispielsweise einen elektrischen Aktuator oder ein Piezoelement, der dazu ausgebildet ist, den Messkörper 16 in radialer Richtung vor und zurück zu bewegen, d. h. radial nach innen in Richtung des Körperteils 106 und radial nach außen vom Körperteil 106 weg (d. h. im Beispiel von Fig. 7 nach unten bzw. oben).

Zusätzlich oder alternativ können die Betätigungsmittel beispielsweise ein oder mehrere verformbare Elemente (nicht gezeigt) umfassen, beispielsweise einen oder mehrere aufblasbare Körper, die beispielsweise so konfiguriert sein können, dass sie verformt werden, um einen Innendurchmesser und/oder einen Umfang des Halteabschnitts 104 zu verringern, z.B. durch Aufblasen des Halteabschnitts 104 oder eines Teils von diesem.

Das Gerät 100 umfasst weiterhin eine Kommunikationseinheit 108, die dazu konfiguriert ist, Überwachungsinformationen bezüglich des Absorptionsgrads der Anregungsstrahlung oder einer Stoffkonzentration, beispielsweise einer Glukosekonzentration, zu übermitteln.

Im Beispiel von Fig. 7 umfasst die Kommunikationseinheit 108 eine optische Anzeige 108A, beispielsweise eine Leuchtdiode (LED).

Der optische Indikator 108A kann beispielsweise dazu konfiguriert sein, Überwachungsinformationen in Bezug auf den Absorptionsgrad der Anregungsstrahlung bereitzustellen, indem er anzeigt, ob ein Glukosespiegel oder eine Glukosekonzentration des Subjekts-/Benutzers, die durch eine oder mehrere Analyt-/Glukosemessungen bestimmt wurde, unter einem vordefinierten Schwellenwert liegt, z. B. unter 70 mg/dl, in einigen Beispielen unter 60 mg/dl, in einem Beispiel unter 50 mg/dl (d. h. um anzuzeigen, ob der Benutzer unter Hypoglykämie leidet oder nicht).

Das Gerät 100 kann beispielsweise eine entsprechende Beschriftung (nicht dargestellt) umfassen, die neben dem optischen Indikator 108A angeordnet ist und die Bedeutung oder Funktion des optischen Indikators 108A angibt oder erklärt (z. B. eine Textbeschriftung "Hypoglykämie" oder "hypoglykämisch").

Die optische Anzeige 108A kann beispielsweise durch dauerhaftes Leuchten oder wiederholtes Blinken anzeigen, dass der Glukosespiegel unter einem vordefinierten Schwellenwert liegt.

In einigen Beispielen kann der optische Indikator 108A auch dazu konfiguriert sein, den Glukosespiegel selbst anzuzeigen, z. B. durch entsprechendes Anpassen der Farbe des vom optischen Indikator 108A emittierten Lichts (z. B. grünes Licht, wenn der Glukosespiegel über einem ersten Schwellenwert liegt, z. B. über 70 mg/dl, gelb, wenn der Glukosespiegel unter dem ersten Schwellenwert, aber über einem zweiten Schwellenwert liegt, z. B. zwischen 50 mg/dl und 70 mg/dl, und rot, wenn der Glukosespiegel unter dem zweiten Schwellenwert liegt, z. B. unter 50 mg/dl).

Der Controller 34 ist außerdem dazu konfiguriert, Überwachungsinformationen in Bezug auf den Absorptionsgrad der Anregungsstrahlung und/oder eine Erste-Hilfe-Anweisung basierend auf dem Absorptionsgrad der Anregungsstrahlung über die Kommunikationseinheit 108 (z. B. durch entsprechende Steuerung der Kommunikationseinheit 108) durch ein oder mehrere der akustischen, optischen und/oder drahtlosen Kommunikationsmittel als Reaktion auf den Bewusstseinszustand bereitzustellen, der anzeigt, dass das Subjekt bewusstlos und/oder nicht ansprechbar ist.

Im Beispiel von Fig. 7 ist der Controller 34 dazu konfiguriert, den optischen Indikator 108A zu aktivieren (und optional eine Farbe des vom optischen Indikator 108A emittierten Lichts anzupassen), wenn der Controller 34 feststellt, dass der Bewusstseinszustand darauf hinweist, dass die Person bewusstlos und/oder nicht ansprechbar ist.

Fig. 8 zeigt eine schematische Darstellung eines Hauptgehäuses 102 einer Vorrichtung zur Überwachung eines Analyten im Gewebe eines menschlichen oder tierischen Subjekts gemäß einer beispielhaften Ausführungsform der Erfindung.

Das Hauptgehäuse 102 kann in einem Gerät gemäß einer der hierin beschriebenen Ausführungsformen verwendet werden, beispielsweise als Hauptgehäuse des Geräts 100 aus Fig. 7.

Das Hauptgehäuse 102 beherbergt eine Vorrichtung zum Nachweis eines Analyten (z. B. Glukose), wie oben beschrieben, einschließlich eines Messkörpers 16, einer Anregungsquelle (nicht dargestellt), einer Nachweisvorrichtung (nicht dargestellt) und einer Steuerung 34, beispielsweise ähnlich dem Gehäuse 24 der Vorrichtung 10 aus Fig. 3.

In anderen Ausführungsformen können einige oder alle Komponenten des Hauptgehäuses 102 in anderen Teilen des Geräts angeordnet sein, mit dem das Hauptgehäuse verwendet wird, beispielsweise im Halteabschnitt 104.

Das Hauptgehäuse 102 umfasst weiterhin eine Kommunikationseinheit 108 zur Bereitstellung von Überwachungsinformationen auf akustischem, optischem und/oder drahtlosem Weg bezüglich des Absorptionsgrads der Anregungsstrahlung und/oder einer Erste-Hilfe-Anweisung basierend auf dem Absorptionsgrad der Anregungsstrahlung.

Im Beispiel von Fig. 8 umfasst die Kommunikationseinheit 108 ein Display 108B (als Beispiel für optische Kommunikationsmittel), das auf oder in einer oberen Oberfläche oder oberen Wand des Hauptgehäuses 102 angeordnet ist, beispielsweise so, dass das Display 108B vom Körperteil 106 weg zeigt, wenn das Gerät, das das Hauptgehäuse 102 umfasst, am Körperteil 106 befestigt ist.

Bei der Anzeige 108B kann es sich beispielsweise um eine Flüssigkristallanzeige und insbesondere um eine organische Leuchtdiodenanzeige (OLED) handeln.

Die Anzeige 108B kann so konfiguriert sein, dass sie das Ergebnis einer oder mehrerer Analyt-/Glukosemessungen anzeigt, beispielsweise den letzten Glukosespiegel und/oder eine Zeitspur des Glukosespiegels.

Die Anzeige 108B kann außerdem dazu konfiguriert sein, Erste-Hilfe-Anweisungen und/oder medizinische Informationen anzuzeigen.

Die Kommunikationseinheit 108 umfasst weiterhin einen Lautsprecher 108C (als Beispiel für akustische Mittel).

Der Lautsprecher 108C ist dazu ausgebildet, Überwachungsinformationen, Erste-Hilfe-Anweisungen und/oder medizinische Informationen wiederzugeben, beispielsweise die jeweiligen Inhalte vorzulesen.

Der Lautsprecher 108C kann auch so konfiguriert sein, dass er einen akustischen Alarm ausgibt.

Die Kommunikationseinheit 108 umfasst außerdem ein drahtloses Kommunikationsmodul 108D, das beispielsweise ein Mobilfunkmodul sein oder umfassen kann, das zum Austauschen von Daten über ein Mobilfunknetz (z. B. ein 4G- und/oder 5G-Mobilfunknetz) konfiguriert ist, und/oder ein drahtloses Netzwerkmodul, das zum Austauschen von Daten über ein drahtloses Netzwerk wie ein Wi-Fi-Netzwerk konfiguriert ist.

Das drahtlose Kommunikationsmodul 108D kann auch derart konfiguriert sein, dass es Daten mit einem externen Gerät wie beispielsweise einem Smartphone des Benutzers austauscht.

Das Hauptgehäuse 102 des Geräts umfasst außerdem eine Vielzahl von Hilfssensoren, die zum Messen von zusätzlichen Parametern konfiguriert sind, die zur Interpretation der Messergebnisse herangezogen werden können.

Im Beispiel von Fig. 8 umfasst das Hauptgehäuse 102 einen Puls- und/oder Sauerstoffsättigungssensor 110, der so konfiguriert ist, dass er einen Puls des Subjekts und/oder einen Sauerstoffsättigungsgrad im Blut des Subjekts bestimmt.

Der Puls- und/oder Sauerstoffsättigungssensor 110 kann beispielsweise ein an sich bekannter optischer Puls- und/oder Sauerstoffsättigungssensor sein.

Der Puls- und/oder Sauerstoffsättigungssensor 210 kann beispielsweise eine oder mehrere Lichtquellen (nicht dargestellt) zum Beleuchten der Haut des Subjekts und einen oder mehrere Fotodetektoren (nicht dargestellt) zum Erfassen von vom Körperteil gestreutem oder reflektiertem Licht umfassen, was zum Bestimmen des Pulses und/oder der Sauerstoffsättigung verwendet werden kann.

Der Puls- und/oder Sauerstoffsättigungssensor 110 kann auf oder in einer Bodenfläche oder Wand des Hauptgehäuses 102 angeordnet sein, z. B. neben dem Messkörper 16, so dass er dem Körperteil 106 zugewandt ist, wenn das Gerät, in dem das Hauptgehäuse 102 verwendet wird, an dem Körperteil befestigt ist.

Das Hauptgehäuse 102 umfasst außerdem einen Beschleunigungssensor oder Beschleunigungsmesser 112, der dazu konfiguriert ist, eine Beschleunigung und/oder eine Richtung der Schwerkraft zu bestimmen.

Der Beschleunigungssensor 112 kann beispielsweise ein piezobasierter Beschleunigungssensor und/oder ein auf einem mikroelektromechanischen System basierender (MEMS-basierter) Beschleunigungssensor sein, wie in der Technik bekannt.

Der Beschleunigungssensor 112 kann beispielsweise dazu ausgebildet sein, eine Beschleunigung entlang zweier oder vorzugsweise dreier orthogonaler Achsen zu ermitteln.

Der Beschleunigungssensor 112 kann so konfiguriert sein, dass er statische (z. B. nicht variierende oder sich langsam ändernde) und dynamische Beiträge zur Beschleunigung trennt, um z. B. eine durch die Schwerkraft verursachte Beschleunigung von einer durch Bewegungen des Benutzers verursachten dynamischen Beschleunigung zu unterscheiden.

Zusätzlich oder alternativ können das Hauptgehäuse 102 und/oder das Gerät 100 auch andere Zusatzsensoren (nicht dargestellt) umfassen, beispielsweise einen Atemsensor, der dazu konfiguriert ist, eine Atemfrequenz des Subjekts und/oder ob das Subjekt atmet, zu bestimmen, und/oder einen Blutdrucksensor, der dazu konfiguriert ist, einen Blutdruck des Subjekts zu bestimmen.

Auch die Atemfrequenz und der Blutdruck sind Beispiele für Parameter, die Aufschluss über den Zustand eines Benutzers geben, beispielsweise, ob die Person bei Bewusstsein und/oder ansprechbar ist.

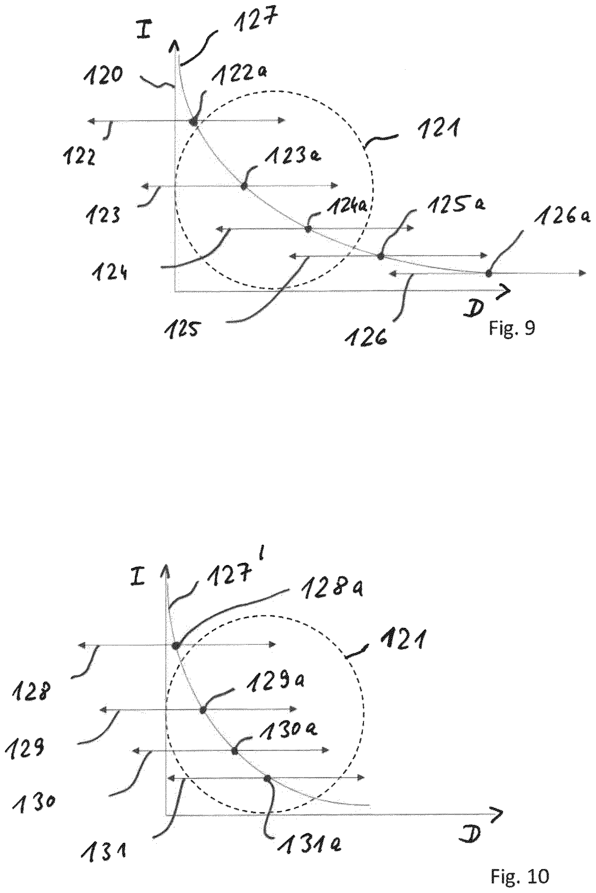

In der Fig. 9 ist schematisch die Ausbreitung einer "thermischen Welle" im Material eines Probekörpers in Richtung zur Oberfläche 120 des Körpers in Abhängigkeit von der Dämpfung der Anregungsstrahlung in dem Probekörper bei einem ersten Absorptionskoeffizienten gezeigt, der bei einer bestimmten ersten Wellenlänge der Anregungsstrahlung vorliegt und kleiner ist als der Absorptionskoeffizient, der der Darstellung in der Figur 10 zugrunde liegt.

Der Durchmesser des eingezeichneten Kreises 121 stellt die Diffusionslänge in dem Probekörper bei einer angenommenen Modulationsfrequenz der Anregungsstrahlung dar. Die Länge der Pfeile 122, 123, 124, 125, 126 entspricht dem Durchmesser des Kreises 121 und zeigt daher, wie weit die thermische Diffusionslänge jeweils von den Punkten 122a, 123a, 124a, 125a, 126a unterhalb der Oberfläche 120 in Richtung zur Oberfläche 120 hin und in der entgegengesetzten Richtung reicht.

Der Intensitätsverlauf I der Anregungsstrahlung in dem Probekörper in Abhängigkeit von der Eindringtiefe D auf der horizontalen Achse ist bei dem bei der ersten Wellenlänge gegebenen Absorptionskoeffizient durch die Kurve 127 dargestellt.

In der Figur 9 zeigt sich, dass bei der eingezeichneten Diffusionslänge hauptsächlich thermische Wellen aus den oberflächennahen Bereichen des Probekörpers, also von den Punkten 122a, 123a an die Oberfläche des Probekörpers gelangen und damit in Form von Temperaturänderungen nachgewiesen oder gemessen werden können. Die durch die Absorption an den Punkten 124a, 125a und 126a ausgesandten thermischen Wellen erzeugen an der Oberfläche keinen großen Temperaturunterschied, da diese Temperaturwellen sich für verschiedene, aufeinander folgende Perioden der modulierten Anregungsstrahlung miteinander vermischen, bevor sie die Oberfläche erreichen. Die Messung bei der in Figur 9 gezeigten Diffusionslänge gibt deshalb Aufschluss über eine Stoffkonzentration in den oberflächennahen Bereichen, in denen die Punkte 122a und 123a liegen.

Wird bei derselben ersten Wellenlänge, das heißt auch derselben Dämpfungscharakteristik, eine weitere Messung mit einer geringeren Modulationsfrequenz und damit mit einer größeren Diffusionslänge durchgeführt, so gelangen auch thermische Wellen von den tiefer liegenden Punkten 124a und 125a a die Oberfläche und können in der Form von separierbaren Temperaturänderungen für jeweils einen Impuls der Anregungsstrahlung nachgewiesen/gemessen werden.

Durch eine Differenzbildung oder eine andere geeignete mathematische Verknüpfung können dann die Beiträge der Messung mit kürzerer Diffusionslänge von den Messergebnissen bei einer größeren Diffusionslänge abgezogen werden und damit kann ein Messergebnis erhalten werden, bei dem die in den oberflächennahen Schichten erhaltenen werte nur noch eine geringe Rolle spielen und damit eine bereinigte Information aus den tieferen Schichten vorliegt. Bei der Messung an menschlicher Haut stammen die oberflächennah gewonnenen Werte aus den abgestorbenen Hautschichten und sind damit für einen aktuellen Glukosewert störend. Durch die beschriebene Kombination Messergebnissen der beschriebenen Messungen können diese störenden Komponenten somit verringert werden.

Fig. 10 zeigt bei einem zweiten Absorptionskoeffizienten, der größer ist als der erste Absorptionskoeffizient, die Ausbreitung einer thermischen Welle im Material eines Probekörpers in Abhängigkeit von der Eindringtiefe der Anregungsstrahlung in den Probekörper.

Die Intensität I der Anregungsstrahlung in dem Probekörper ist auf der vertikalen Achse in Abhängigkeit von der Eindringtiefe D ist auf der horizontalen Achse bei dem bei einer zweiten Wellenlänge gegebenen Absorptionskoeffizient durch den Intensitätsverlauf/die Kurve 127' dargestellt. Der für die Kurve 127' zugrunde gelegte Absorptionskoeffizient ist größer als der für die Kurve 127 der Figur 9 zugrunde gelegte Absorptionskoeffizient, so dass die Intensität der Anregungsstrahlung in dem Probekörper mit zunehmender Eindringtiefe schneller abnimmt.

Da die gesamte Energie der Anregungsstrahlung oder zumindest ihr wesentlicher Teil aufgrund der starken Dämpfung bereits in sehr oberflächennahen Schichten absorbiert wird, stammen, wenn dieselbe Modulationscharakteristik und damit dieselbe Diffusionslänge wie bei der der Figur 9 zugrundeliegenden Messung angewandt wird, auch die an der Oberfläche 120 ankommenden thermischen Wellen im Wesentlichen aus den oberflächennahen Schichten. Um in stärkerem Maß thermische Wellen aus tieferen Schichten an der Oberfläche nachweisen zu können, kann beispielsweise bei diesem Absorptionskoeffizienten und der entsprechenden Wellenlänge der Anregungsstrahlung eine höhere Intensität der Anregungsstrahlung gewählt werden. Weiter ist es sinnvoll, bei der Messung, bei der der Schwerpunkt auf den oberflächennahen Schichten liegt und durch die Modulationscharakteristik eine kürzere Diffusionslänge eingestellt wird, eine noch kürzere Diffusionslänge einzustellen als bei der Messung bei einer Wellenlänge mit einem kleineren Absorptionskoeffizienten. Auch die Messung der tieferen Schichten kann bei einer geringeren Diffusionslänge durchgeführt werden, als sie bei einer Messung bei einer Wellenlänge mit kleinerem Absorptionskoeffizienten angewandt wird, um nicht alle thermischen Impulse aus der Tiefe der Probe mitzumessen, da in diesem Fall das Messergebnis unabhängig von der Analytkonzentration in der Probe wird (Sättigung)..Je nach den vorliegenden physiologischen Verhältnissen kann mit steigender Dämpfung in dem zu vermessenden Gewebe für die Tiefenmessung eine Verlängerung der thermischen Diffusionslänge, also eine Verringerung der Modulationsfrequenz, oder eine Verkürzung der thermischen Diffusionslänge, also eine Vergrößerung der Modulationsfrequenz sinnvoll sein. Durch Kalibrierungsmessungen bei Nutzern der Messeinrichtung kann eine Zuordnungskurve aufgenommen werden, die den jeweils wellenlängenabhängig gemessenen Dämpfungswerten Modulationscharakteristiken für die Tiefenmessungen (also für eine betreffende Wellenlänge für diejenigen Messungen mit der größeren oder größten thermischen Diffusionslänge) zuordnet.

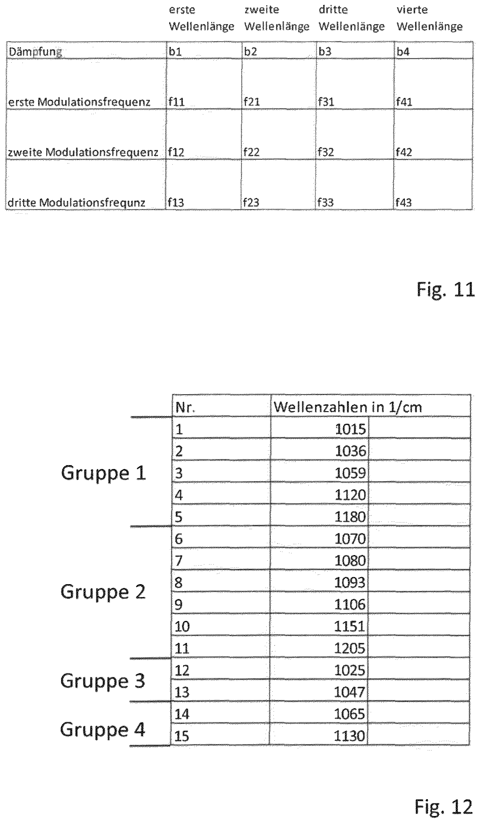

Fig. 11 zeigt eine Tabelle von verschieden starken Dämpfungen b1, b2, b3, b4, einer Anregungsstrahlung bei der Messung von Glukose an menschlicher Haut, die beispielsweise durch verschieden große Absorptionskoeffizienten für verschiedene Wellenlängen bedingt sein können, jedoch teilweise auch zusätzlich durch Oberflächenbeläge mit stark absorbierendem Material verschiedener Dicke, wie beispielsweise bei Individuen verschieden dicke Hornhaut oder durch einen Laktatbelag.

Wird angenommen, dass jede der vier Spalten eine andere Wellenlänge/Wellenzahl der Anregungsstrahlung betrifft, so ergeben sich in dem Beispiel vier verschiedene Dämpfungen für unterschiedliche Wellenlängen/Wellenzahlen, die wenigstens teilweise auch vier verschiedenen Absorptionskoeffizienten bei den verschiedenen Wellenlängen/Wellenzahlen entsprechen können.

In jeder der Spalten sind beispielhaft drei Modulationscharakteristiken angegeben, beispielsweise für die erste Wellenzahl bei einer angenommenen periodischen Modulation die Modulationsfrequenzen f11, f12 und f13, und für die zweite Wellenzahl die Modulationsfrequenzen f21, f22 und f23.

Oft werden bei einer Wellenzahl auch nur zwei oder im Extremfall auch nur eine einzige Modulationscharakteristik angewendet.

Es wird angenommen, dass die Modulationsfrequenzen f11, f21, f31, f41 der obersten Reihe die höchsten für die jeweiligen Wellenzahlen sind und somit bei jeder der Wellenzahlen jeweils die kürzeste thermische Diffusionslänge erzeugen, dass die Modulationsfrequenzen f13, f23, f33 und f43 der untersten Reihe die niedrigsten sind und die größten thermischen Diffusionslängen erzeugen und dass die Modulationsfrequenzen f12, f22, f32 f42 der mittleren Reihe zwischen denen der oberen und den Modulationsfrequenzen der untersten Reihe liegen.

Dann würden, wenn die Dämpfung b1 größer ist als die Dämpfung b2 und die Dämpfung b2 größer ist als die Dämpfung b3 in den meisten Fällen die Modulationsfrequenz f13 höher gewählt als die Modulationsfrequenz f23 und diese höher als die Modulationsfrequenz f33. Auch die Modulationsfrequenzen mit den zweithöchsten Diffusionslängen f12, f22 und f32 könnten in manchen Fällen entsprechend gewählt werden, so dass f12 größer ist als f22 und diese größer als f32, sofern bei jeder Wellenzahl mit drei verschiedenen Modulationsfrequenzen gemessen werden soll.

Die Figur 12 zeigt eine Auswahl von Wellenzahlen, jeweils angegeben in der Einheit 1/cm, die für eine nichtinvasive Glukosemessung sinnvoll sein können, weil das Glukosemolekül bei diesen Wellenzahlen eine ausreichende Absorption zeigt und gleichzeitig bei typischen Gewebezusammensetzungen an diesen Stellen das Absorptionsspektrum der Glukose nicht vollständig von Absorptionsbereichen anderer Stoffe wie Wasser, Laktat und anderen Stoffen, überdeckt wird. Die Lage der hier aufgeführten Wellenzahlen im Spektrum ist aus der Betrachtung der Fig. 2 erkennbar. Dabei können die optimalen Wellenzahlen in vielen Fällen auch etwas oberhalb oder unterhalb dieser Wellenzahlen angeordnet sein, weshalb auch im Rahmen dieses Textes teilweise Wellenzahlbereiche um die genannten Wellenzahlen herum angegeben sind. Manche Wellenzahlen können unter bestimmten Umgebungsbedingungen auch grundsätzlich ungeeignet für eine Messung sein. Die optimierte Auswahl der Wellenzahlen kann vor einer Glukosemessung in vielen Fällen durch eine Vorbereitungsmessung bei dem Nutzer ermittelt werden, indem beispielsweise eine oberflächennahe Messung mit hohen Modulationsfrequenzen, beispielsweise bei mehr als 1khz oder mehr als 1,3khz, durchgeführt wird, um die Absorptionskoeffizienten und/oder die Dämpfung wellenzahlabhängig für den individuellen Fall zu messen.

Zur besseren Beschreibung sind die angegebenen Wellenzahlen in der Fig. 12 durchnummeriert und auf diese Nummerierung wird im Folgenden Bezug genommen.

Es hat sich gezeigt, dass die erste Gruppe von Wellenzahlen mit den Nr. 1-5 diejenigen sind, die in den meisten Fällen von hoher Wichtigkeit und Aussagekraft bei den Messungen sind. Aus diesen können fünf, vier, drei oder zwei oder in seltenen Fällen auch nur eine Wellenzahl für die Messung ausgewählt werden. In vielen Fällen werden dann noch eine, zwei, drei oder mehr Wellenzahlen aus der zweiten Gruppe hinzugefügt, die die Wellenzahlen mit den Nr. 6-11 enthält. In manchen Fällen kann es auch nützlich sein, noch eine oder zwei Wellenzahlen aus der dritten Gruppe mit den Nummern 12 und 13 hinzuzufügen und/oder eine oder zwei der Wellenzahlen 14 und 15, die die vierte Gruppe bilden.

Grundsätzlich kann bei einer Messung für die Wellenzahlen der ersten Gruppe eine höhere Laserleistung angewendet werden als für die Wellenzahlen der übrigen Gruppen und für die Wellenzahlen der zweiten Gruppe eine höhere Laserleistung als für die dritte und vierte Gruppe. E ist sinnvoll, insgesamt für eine gut auswertbare Messung zwischen 5 und 15 Wellenzahlen auszuwerten, vorzugsweise 8-13 Wellenzahlen.

Weiter kann es sinnvoll sein, dass wenigstens die Hälfte oder zwei Drittel oder wenigstens 80 Prozent der verwendeten Wellenzahlen aus der ersten Gruppe ausgewählt werden.

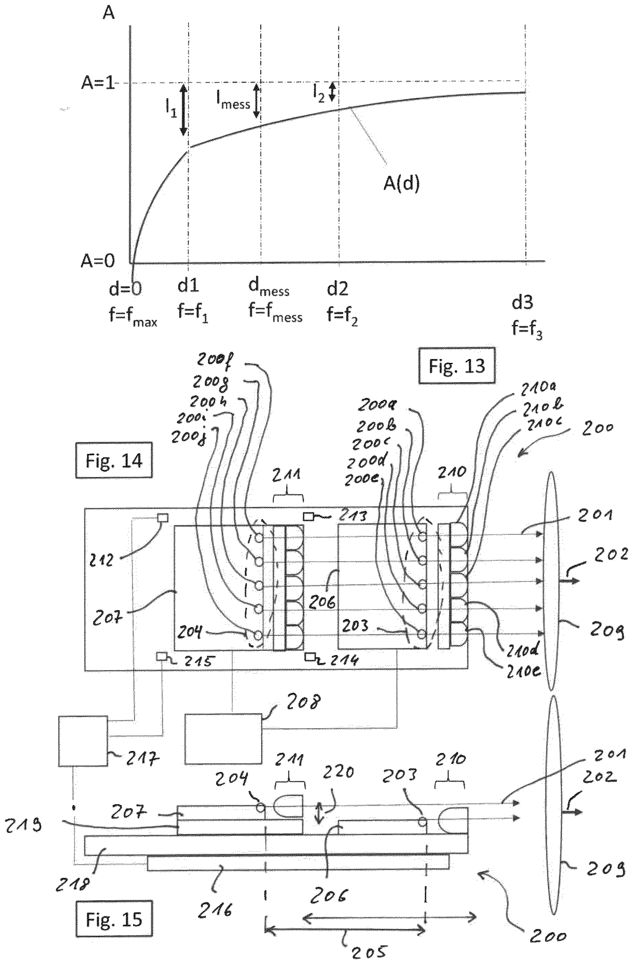

Die Figur 13 zeigt ein Koordinatensystem, bei dem auf der horizontalen Achse die Tiefe d im Messvolumen unterhalb der Oberfläche aufgetragen ist. Der Punkt d=0 entspricht dabei der Oberfläche.

Auf der vertikalen Achse ist der bis zu der jeweiligen Tiefe d bereits in dem Volumen absorbierte Anteil Ader Anregungsstrahlung aufgetragen. Die Funktion A (d) ist demnach eine Repräsentation der Dämpfungskurve. In einer großen Tiefe ist A=1 asymptotisch erreicht, das heißt, bis dorthin ist die gesamte Anregungsstrahlung absorbiert.

Die Tiefe d1 soll der unteren Grenze des Stratum Corneum entsprechen. Bis dorthin, das heißt innerhalb des Stratum Corneum, ist in dem Beispiel ein relativ großer Absorptionskoeffizient gegeben, so dass dort die Funktion A (d) relativ steil ist. Von der Tiefe d1 an ist ein geringerer Absorptionskoeffizient gegeben, so dass von der Tiefe d1 an ein flacherer Funktionsverlauf A(d) und im Punkt A(d1) eine deutliche Steigungsänderung in dem Funktionsgraphen gegeben ist. Durch die Lage des Punktes A(d1) und den Verlauf von A(d) für geringere Tiefen kann die Funktion A(d) in vielen Fällen bereits weitgehend charakterisiert werden. Es kann in vielen Fällen auch sinnvoll sein, noch Messwerte für d>d1 für die Charakterisierung hinzuzunehmen.

Die mit $I_1$, $I_{mess}$ und $I_2$ gekennzeichneten Doppelpfeile zeigen an den jeweiligen Punkten der Tiefe $d_1$, $d_{mess}$ und $d_2$ den noch verbleibenden, in der jeweiligen Tiefe noch nicht absorbierten Anteil der eingestrahlten Intensität der Anregungsstrahlung an, der gebildet ist durch den Abstand der Funktion A(d) von der Asymptoten, die der Dämpfung A=1 und der verbleibenden Intensität Null entspricht. Eine Tiefe $d_{mess}$, in der oder bis zu der sinnvollerweise durch die Wahl der entsprechenden Modulationsfrequenz $f_{mess}$ die Antwortsignale substanziell gewichtet gemessen werden, kann vorteilhaft so gewählt werden, dass $d_{mess}$, größer ist als die Dicke des Stratum Corneum und so klein, dass $I_{mess}$, also der bis zu dieser Tiefe noch nicht absorbierte Anteil der Anregungsstrahlung, mehr als 1%, insbesondere mehr als 10%, weiter insbesondere mehr als 20%, weiter insbesondere mehr als 30% der Intensität der Anregungsstrahlung beim Eintritt in das Messvolumen/die Probe /das Gewebe beträgt.

Mit $I_2$ ist die verbleibende Restintensität der Anregungsstrahlung in der Tiefe $d_2$ bezeichnet, wobei diese Tiefe einer Modulationsfrequenz f3 entspricht und wobei angenommen wird, dass $d_2$ die größte Tiefe ist, in der eine Messung noch sinnvoll unterhalb der Sättigung durchgeführt werden kann.

In der Tiefe $d_3$ wird angenommen, dass dort praktisch keine Intensität der Anregungsstrahlung mehr hingelangt. Das bedeutet, wenn die Modulationsfrequenz $f_3$ gewählt wird, wird unabhängig von der Konzentration des nachzuweisenden Stoffes die Absorption der gesamten Anregungsstrahlung im Messvolumen nachgewiesen.

Zur Erklärung soll der Fall einer zeitlich periodischen Modulation der Intensität der Anregungsstrahlung betrachtet werden. Innerhalb einer Periode der Modulation gelangen jeweils Antwortsignale aus einem bestimmten Tiefenbereich des Volumens, in das die Anregungsstrahlung eingestrahlt wird und in dem die Stoffkonzentration gemessen werden soll, bis an die Oberfläche des Volumens oder des Gewebes. Je größer die Periodendauer der Modulation, also, je geringer die Modulationsfrequenz ist, umso größer ist die Tiefe im Messvolumen unterhalb der Oberfläche, aus der die Antwortsignale, beispielsweise Druckwellen oder Temperatursignale innerhalb der Periode an die Oberfläche gelangen und gemessen werden können. Somit lässt sich die maximale Messtiefe durch die Wahl der Modulationsfrequenz bestimmen. Für die Intensität der gemessenen Antwortsignale spielt zusätzlich die Dämpfung der Anregungsstrahlung innerhalb des Messvolumens/Gewebes eine wichtige Rolle. Je stärker die Anregungsstrahlung bei Erreichen einer bestimmten Tiefe unter der Oberfläche bereits durch Absorption reduziert ist, umso geringer ist die nachweisbare Reaktion in dieser Tiefe.

Wird eine sehr geringe Modulationsfrequenz gewählt, so wird praktisch die Reaktion auf die gesamte Anregungsstrahlung im gesamten Messvolumen nachgewiesen und das erfasste Signal ist gesättigt und unabhängig von der Tiefe, in der die Anregungsstrahlung absorbiert wurde und somit auch unabhängig von der Konzentration des nachzuweisenden Stoffes in dem Volumen. Es ist daher sinnvoll, eine Frequenz der Modulation zu wählen, die einer maximale Tiefe der nachgewiesenen Reaktionen entspricht, die um einen deutlichen Betrag geringer ist als die Tiefe, in der die Sättigung erreicht ist Beispielsweise kann die Modulationsfrequenz, nachdem die Dämpfungsfunktion in dem Volumen ermittelt wurde, so gewählt werden, dass die maximale Tiefe, aus der innerhalb der Periode der Modulation Antwortsignale an die Oberfläche gelangen, so bemessen ist, dass bis zu dieser Tiefe nicht mehr als 70% oder nicht mehr als 80% oder nicht mehr als 90% oder nicht mehr als 98 oder nicht mehr als 99% der Anregungsstrahlung absorbiert sind.

Diese Angabe der maximalen Tiefe kann beispielsweise in einer möglichen Interpretation so verstanden werden, dass aus dieser Tiefe innerhalb der Periode der Modulation eine substanzielle Wärmemenge bis an die Oberfläche übertragen wird, beispielsweise wenigstens 10%, wenigstens 20% oder wenigstens 30% der in der Tiefe freigesetzten Wärmemenge.

Die Dämpfungsfunktion und der Verlauf der verbleibenden Intensität der Anregungsstrahlung in Abhängigkeit von der Tiefe unter der Oberfläche des Volumens/Gewebes, sind durch den Absorptionskoeffizienten des Materials in dem Volumen bestimmt. Dieser Absorptionskoeffizient ist abhängig von der Wellenlänge der Anregungsstrahlung. Zudem liegt oft in dem Volumen eine Kombination von übereinanderliegenden Schichten vor, die jeweils verschiedene Absorptionskoeffizienten aufweisen. Die Dämpfung ergibt sich somit aus einer Kombination von Schichtdicken und Absorptionskoeffizienten der Schichten. Wird beispielsweise in der menschlichen Haut gemessen, so hängt die Dämpfungsfunktion von der Dicke der verschiedenen Hautschichten und ihren individuellen Absorptionskoeffizienten ab. Die oberste Hautschicht, das stratum corneum, weist beispielsweise auch in Abhängigkeit von ihrem individuellen Wassergehalt, einen relativ hohen Absorptionskoeffizienten auf, der zudem durch einen variierenden Wassergehalt auch stark variieren kann. Um die wellenlängenabhängigen Modulationscharakteristiken zu ermitteln, kann deshalb zunächst der Dämpfungsverlauf der Anregungsstrahlung bei der jeweiligen Wellenlänge in Abhängigkeit von der Tiefe im Volumen/Messvolumen ermittelt werden.

Zu diesem Zweck wird die Dämpfung an einer bestimmten Anzahl von Stützstellen, das heißt in verschiedenen Tiefen des Volumens, gemessen, die durch bestimmte Modulationscharakteristiken, beispielsweise bestimmte Modulationsfrequenzen gegeben sind. Mit diesen Stützstellen kann die Dämpfungsfunktion ermittelt und danach können unter Berücksichtigung der Dämpfungsfunktion die für die eigentliche Messung der Stoffkonzentration möglichen oder optimierten Modulationscharakteristiken/Modulationsfrequenzen festgelegt werden.

Es wurde gefunden, dass in manchen Fällen mehr als 3, mehr als 4, mehr als 5 oder mehr als 7 verschiedene Modulationscharakteristiken ausreichen, um die Dämpfungsfunktion ausreichend zu charakterisieren.

Eine solche Charakterisierung der Dämpfungsfunktion gelingt jedoch nicht nur unter Verwendung von periodischen Modulationen, sondern auch mit anderen Modulationscharakteristiken, beispielsweise impulsförmigen Modulationen, da diesen im Frequenzraum durch eine Fourier - Transformation bestimmte Überlagerungen von Modulationsfrequenzen zugeordnet werden können.

In den Figuren 14 und 15 ist eine Anregungseinrichtung mit einer Anregungsquelle 200 schematisch dargestellt, und zwar in der Figur 14 von oben gesehen und in der Figur 15 von einer Seite gesehen.

Die Anregungsquelle 200 weist zwei Halbleiterwafer 206, 207 auf, von denen jeder 5 Anregungsquellenelemente 200a, 200b, 200c, 200d, 200e, 200f, 200g, 200h, 200i, 200j in der Form von Quantenkaskadenlasern aufweist. Die Quantenkaskadenlaser sind jeweils äquidistant in einer Reihe entlang einer geraden Linie am Rand jedes der Halbleiterwafer 206, 207 angeordnet. Die einzelnen Quantenkaskadenlaser strahlen jeweils Licht in ihren individuellen Wellenlängen oder schmalen Wellenlängenbereichen in einer allen Quantenkaskadenlasern gemeinsamen Strahlrichtung ab, die durch den Pfeil 202 angezeigt ist. Es sind Gruppen 203, 204 von Quantenkaskadenlasern gebildet, wobei die erste Gruppe 203 die Anregungsquellenelemente/Quantenkaskadenlaser 200a, 200b, 200c, 200d, 200e und die zweite Gruppe 204 die Anregungsquellenelemente/Quantenkaskadenlaser 200f, 200g, 200h, 200i, 200j umfasst. Jede Gruppe kann beispielsweise zwischen 2 und 10 Quantenkaskadenlasern umfassen und es können auch mehr als 2 Gruppen von Quantenkaskadenlasern vorgesehen sein, die voneinander in der Strahlrichtung 202 beanstandet sind. Der Abstand zwischen der ersten und der zweiten Gruppe ist in der Figur durch den Pfeil 205 angezeigt und kann zwischen 3 und 7 mm, insbesondere zwischen 4 und 6 mm betragen. Der Abstand der beiden Gruppen 203, 204 von Quantenkaskadenlasern zueinander in Richtung senkrecht zur Strahlrichtung 202 und senkrecht zum Verlauf der Reihen von Quantenkaskadenlasern in Richtung des Pfeils 220 kann beispielsweise 500 $\mu$m betragen. Im oberen Teil der Figur sind 4 NTC -Elemente 212, 213, 214, 215 dargestellt, die über Messleitungen mit einer Temperatursteuereinheit 217 verbunden sind. Durch die NTC -Elemente wird die Temperatur in verschiedenen Bereichen der Halbleiterwafer 206, 207 gemessen um die Kühlung/Entwärmung zu steuern, die durch wenigstens ein Peltierelement 216 geleistet oder zumindest unterstützt wird. Das Peltierelement 216 ist unterhalb einer Trägerplatte 218 angeordnet, die die Halbleiterwafer 206, 207 trägt. Wie in der Figur 15 ersichtlich ist, sind die beiden Halbleiterwafer 206, 207, die jeweils eine Gruppe von Quantenkaskadenlasern tragen oder umfassen, ebenso wie die Quantenkaskadenlaser selbst um die durch den Pfeil 205 repräsentierte Strecke in der Strahlrichtung 202 beanstandet. Jedem der Halbleiterwafer 206,207 kann auch ein einzelnes Peltierelement 216 zugeordnet sein, sodass die Kühlung der einzelnen Halbleiterwafer unabhängig voneinander gesteuert werden kann. Die Kühlleistung kann damit besser konzentriert und somit effektiver genutzt werden. Die Aufteilung der Wellenlängen der einzelnen Quantenkaskadenlaser kann derart vorgenommen werden, dass eine erste Gruppe 203 von Quantenkaskadenlasern ersten Wellenbereich abdeckt und eine zweite Gruppe 204 von Quantenkaskadenlasern einen zweiten Wellenlängenbereich, der sich nicht oder nur in geringem Maße mit dem Wellenlängenbereich der ersten Gruppe überschneidet. Dadurch kann im Herstellungsverfahren, dass im Regelfall ein Epitaxieverfahren ist, jeder der Halbleiterwafer in Bezug auf die Wellenlängen der Quantenkaskadenlaser, die er trägt bzw. die in ihn integriert sind, herstellungstechnisch optimiert werden.

In dem gezeigten Beispiel sind die Linien, auf denen die Quantenkaskadenlaser jeweils einer Gruppe 203, 204 angeordnet sind als gerade Linien entlang den Rändernder Halbleiterwafer gebildet, die senkrecht auf der Strahlrichtung 202 stehen und zueinander parallel sind. Eine weitere Gruppe von Quantenkaskadenlasern könnte beispielsweise in einer Linie auf einem weiteren Halbleiterwafer vom Halbleiterwafer 206 aus gesehen hinter dem Halbleiterwafer 207 angeordnet sein.

Die Halbleiterwafer 206, 207 sind derart angeordnet, dass die Gruppe 204 von Quantenkaskadenlasern von der Trägerplatte 218 durch einen Zwischenlagekörper 219 ein Stück weit beanstandet ist, während der Halbleiterwafer 206 unmittelbar auf der Trägerplatte 218 montiert ist. Damit ergibt sich ein Versatz der Gruppe 203 von Quantenkaskadenlasern gegenüber der Gruppe 204 von Quantenkaskadenlasern um beispielsweise 500 Mikrometer in Richtung des in der Figur 15 eingezeichneten Doppelpfeils 220 senkrecht zur Strahlrichtung 202, sodass die Anregungsstrahlung der Gruppe 204 über die Quantenkaskadenlaser der Gruppe 203 hinweggehen kann.

Die Anordnung der beiden Reihen von Quantenkaskadenlasern übereinander ermöglicht es, zusammen mit einer begrenzten Länge der jeweiligen Reihe von Quantenkaskadenlasern die Anregungsstrahlen, die durch die einzelnen Quantenkaskadenlaser ausgesandt werden, alle in einem relativ geringen Abstand von einer zentralen Strahlachse zu halten. Auf diese Weise können alle Strahlen durch eine gemeinsame Makrolinse 209 geführt, beispielsweise kollimiert oder fokussiert werden.

Beim Austritt aus den Anregungsquellenelementen/Quantenkaskadenlasern wird die Anregungsstrahlung jedes einzelnen Quantenkaskadenlasers in einer Mikrolinse 210a, 210b, 210c, 210d, 210e geformt oder kollimiert. Jede Mikrolinse für sich weist zu diesem Zweck 2 azirkular -zylindrische, voneinander abgewandte Begrenzungsflächen auf, wobei die Zylinderachsen der beiden Begrenzungsflächen senkrecht aufeinander stehen. Die Mikrolinsen, die jeweils einer der Gruppen von Quantenkaskadenlasern zugeordnet sind, bilden je Gruppe ein zusammenhängendes Mikrolinsenarray 210, 211. Die jeweils zwei Begrenzungsflächen jeder Mikrolinse sind jeweils einer der optischen Achsen des Halbleiters zugeordnet, in dem die Strahlung erzeugt wird und sie sind für die Kollimierung bezüglich jeweils einer Linearpolarisationsrichtung zuständig. Die Konizität der den Quantenkaskadenlasern unmittelbar zugewandten Zylinderfläche kann dabei zwischen -10 und -14, insbesondere etwa -12 betragen. Diese durchgehende, allen Mikrolinsen gemeinsame Zylinderfläche ist für die Kollimierung der sogenannten schnellen Achse (FAC) der Strahlung zuständig. Auf der den Quantenkaskadenlasern abgewandten Seite der Mikrolinsen sind nebeneinanderliegende, jeweils einem Quantenkaskadenlaser zugeordnete azirkular - zylindrische Flächen mit zueinander parallelen Zylinderachsen vorgesehen, die jeweils die Strahlung bezüglich der sogenannten langsamen Achse kollimieren. Die Konizität der den Quantenkaskadenlasern abgewandten azirkular -zylindrischen Flächen kann zwischen -2 und -4, insbesondere -3 betragen.

Die auf diese Weise gebildeten Mikrolinsen können durch ein Epitaxieverfahren auf einem Halbleiterwafer hergestellt werden. Die Dicke der so hergestellten doppelt azirkular -zylindrischen Linsen kann etwa 500 $\mu$m betragen und richtet sich nach der Dicke des Halbleiterwafers.

Durch die beschriebene Kombination von Mikrolinsen und Makrolinse können die Spots der verschiedenen Quantenkaskadenlaser in einen gemeinsamen Targetbereich gebracht werden und die Anregungsstrahlen der einzelnen Quantenkaskadenlaser können weitestgehend zur Deckung gebracht werden, wobei gleichzeitig die Form des Laserspots kompakt gehalten und in der richtigen Form und Größe gestaltet werden kann.

Die in der Figur 14 gezeigte Ansteuereinrichtung 208 ist dazu eingerichtet, die einzelnen Quantenkaskadenlaser aufeinander abgestimmt anzusteuern und ihre Intensität, duty cycle sowie eine Modulationsfrequenz zu steuern.

Die Quantenkaskadenlaser können derart eingerichtet sein, dass sie Strahlung im mittleren Infrarotbereich abstrahlen.

Obwohl die vorliegende Erfindung im Hinblick auf spezifische Ausführungsformen beschrieben wurde, versteht es sich, dass dem Fachmann Variationen und Modifikationen einfallen, die alle als Aspekte der vorliegenden Erfindung gedacht sind.

Dementsprechend sollten für die Erfindung nur die in den Ansprüchen aufgeführten Beschränkungen gelten.

**Patentansprüche**

1. Anregungsquelle (26, 200) zur Einstrahlung von Anregungsstrahlung (18, 201) in ein Volumen, mit einer Mehrzahl von Anregungsquellenelementen (200a, 200b, 200c, 200d, 200e, 200f, 200g, 200h, 200i, 200j), von denen jedes eine Anregungsstrahlung (18, 201) in einem ihm zugeordneten Wellenlängenbereich in einer gemeinsamen Strahlrichtung (202) aussendet, **dadurch gekennzeichnet, dass** wenigstens eine erste und eine zweite Gruppe (203, 204) mit jeweils mehreren Anregungsquellenelementen vorgesehen sind, wobei die Anregungsquellenelemente jeder Gruppe auf einer gemeinsamen, quer zur Strahlrichtung, insbesondere senkrecht zur Strahlrichtung, angeordneten Fläche oder Linie angeordnet sind und wobei die verschiedenen Gruppen von Anregungsquellenelementen in Strahlrichtung voneinander beabstandet sind.

2. Anregungsquelle nach Anspruch 1, **dadurch gekennzeichnet, dass** wenigstens eine erste und eine zweite Gruppe von Anregungsquellenelementen (200a, 200b, 200c, 200d, 200e, 200f, 200g, 200h, 200i, 200j) jeweils entlang von senkrecht zur Strahlrichtung (202) verlaufenden Linien angeordnet sind, **dadurch gekennzeichnet, dass** die Linien dieselbe Form aufweisen und gegeneinander in Richtung senkrecht zur Strahlrichtung versetzt sind.

3. Anregungsquelle nach einem der Ansprüche 1 oder 2, **dadurch gekennzeichnet, dass** die Linien zueinander abschnittsweise parallel verlaufen.

4. Anregungsquelle nach Anspruch 2 oder 3, **dadurch gekennzeichnet, dass** die Linien gerade Linien sind.

5. Anregungsquelle nach einem der Ansprüche 1 bis 4, **dadurch gekennzeichnet, dass** die Anregungsquellenelemente (200a, 200b, 200c, 200d, 200e, 200f, 200g, 200h, 200i, 200j) Quantenkaskadenlaser sind.

6. Anregungsquelle nach einem der Ansprüche 1 bis 5, **dadurch gekennzeichnet, dass** jede der Gruppen (203, 204) von Anregungsquellenelementen (200a, 200b, 200c, 200d, 200e, 200f, 200g, 200h, 200i, 200j) auf jeweils einem separaten Halbleiterwafer (206, 207) angeordnet ist.

7. Anregungsquelle nach einem der Ansprüche 1 bis 6, **dadurch gekennzeichnet, dass** wenigstens zwei der separaten Halbleiterwafer (206, 207), auf denen jeweils eine Gruppe (203, 204) von Anregungsquellenelementen (200a, 200b, 200c, 200d, 200e, 200f, 200g, 200h, 200i, 200j) angeordnet ist, miteinander fest verbunden sind.

8. Anregungsquelle nach einem der Ansprüche 1 bis 7, **dadurch gekennzeichnet, dass** eine gemeinsame Ansteuereinrichtung (208) vorgesehen ist, die eine Mehrzahl von Anregungsquellenelementen (200a, 200b, 200c, 200d, 200e, 200f, 200g, 200h, 200i, 200j) ansteuert.

9. Anregungsquelle nach einem der Ansprüche 1 bis 8, **dadurch gekennzeichnet, dass** eine gemeinsame Makrolinse (209) zur Strahlformung der Anregungsstrahlung (18, 201) von mehreren oder allen Anregungsquellenelementen (200a, 200b, 200c, 200d, 200e, 200f, 200g, 200h, 200i, 200j) einer Gruppe (203, 204) oder mehrerer verschiedener, in Strahlrichtung (202) voneinander beabstandeter Gruppen vorgesehen ist.

10. Anregungsquelle nach einem der Ansprüche 1 bis 9, **dadurch gekennzeichnet, dass** für wenigstens eine der Gruppen (203, 204) von Anregungsquellenelementen (200a, 200b, 200c, 200d, 200e, 200f, 200g, 200h, 200i, 200j) eine separate Mikrolinsenanordnung (210, 211) vorgesehen ist.

11. Anregungsquelle nach Anspruch 10, **dadurch gekennzeichnet, dass** wenigstens einer der Gruppen (203, 204) von Anregungsquellenelementen (200a, 200b, 200c, 200d, 200e, 200f, 200g, 200h, 200i, 200j) Mikrolinsen (210a, 210b, 210c, 210d, 210e, 210f, 210g, 210h, 210i, 210j) zugeordnet sind, die eine azirkular-zylindrische Form aufweisen und von denen jeweils eine vor einem der Anregungsquellenelemente angeordnet ist.

12. Anregungsquelle nach einem der Ansprüche 10 oder 11, **dadurch gekennzeichnet, dass** jede der Mikrolinsen zwei voneinander abgewandte, jeweils azirkular-zylindrische Außenflächen aufweist.

13. Anregungsquelle nach einem der Ansprüche 10 bis 12, **dadurch gekennzeichnet, dass** jede Anordnung von Mikrolinsen, die einer Gruppe von Anregungsquellenelementen (200a, 200b, 200c, 200d, 200e, 200f, 200g, 200h, 200i, 200j) zugeordnet ist, als Mikrolinsenarray ausgebildet ist, in dem die Mikrolinsen fest miteinander verbunden sind.

14. Anregungsquelle nach einem der Ansprüche 1 bis 13, **dadurch gekennzeichnet, dass** alle oder wenigstens 80 % der Anregungsquellenelemente (200a, 200b, 200c, 200d, 200e, 200f, 200g, 200h, 200i, 200j) einer ersten Gruppe (203, 204) eine größere Wellenlänge aufweisen als alle Anregungsquellenelemente einer zweiten Gruppe (203, 204).

15. Vorrichtung zum Nachweis eines Stoffes, insbesondere zur Messung einer Stoffkonzentration, in einem Volumen, insbesondere einer Probe oder einem Gewebe, mit einer Anregungsquelle (26, 200) nach einem der Ansprüche 1 bis 14 zur Einstrahlung einer Anregungsstrahlung (18, 201) verschiedener Wellenlängen in das Volumen und mit einer Detektionseinrichtung (16) zur Erfassung eines durch die Absorption der Strahlung in dem Volumen oder dem Gewebe oder der Probe erzeugten Antwortsignals in Abhängigkeit von der Wellenlänge der Anregungsstrahlung, sowie mit einer Modulationseinrichtung zur Modulation des Verlaufs der Intensität der Anregungsstrahlung.

**Fig. 1**

IR spectra of glucose at different concentrations

**Fig. 2**

**Fig. 3**

Clarke's Error Grid Analysis-raw Amplitude

1558 Datenpunkte

20 Probanden davon
17 Gesunde,
2 Typ 1 Diabetiker
1 Typ 2 Diabetiker

Zone A:95,4%
Zone B:4,0%
Zone C:0%
Zone D:0,6%
Zone E:0%

**Fig. 4**

**Fig. 5**

**Fig. 6**

Fig. 7

Fig. 8

Fig. 9

Fig. 10

|  | erste Wellenlänge | zweite Wellenlänge | dritte Wellenlänge | vierte Wellenlänge |
|---|---|---|---|---|
| Dämpfung | b1 | b2 | b3 | b4 |
| erste Modulationsfrequenz | f11 | f21 | f31 | f41 |
| zweite Modulationsfrequenz | f12 | f22 | f32 | f42 |
| dritte Modulationsfrequnz | f13 | f23 | f33 | f43 |

Fig. 11

|  | Nr. | Wellenzahlen in 1/cm |
|---|---|---|
| Gruppe 1 | 1 | 1015 |
| | 2 | 1036 |
| | 3 | 1059 |
| | 4 | 1120 |
| | 5 | 1180 |
| Gruppe 2 | 6 | 1070 |
| | 7 | 1080 |
| | 8 | 1093 |
| | 9 | 1106 |
| | 10 | 1151 |
| | 11 | 1205 |
| Gruppe 3 | 12 | 1025 |
| | 13 | 1047 |
| Gruppe 4 | 14 | 1065 |
| | 15 | 1130 |

Fig. 12

Fig. 13

Fig. 14

Fig. 15

**Europäisches Patentamt**
**European Patent Office**
**Office européen des brevets**

# EUROPÄISCHER RECHERCHENBERICHT

**Nummer der Anmeldung**

EP 24 19 2874

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 11 881 678 B1 (BISHOP MICHAEL J [US] ET AL) 23. Januar 2024 (2024-01-23) | 1-14 | INV. |
| Y | * Abbildungen 1, 2B, 2C, 4A, 4B, 8 * | 15 | H01S5/34 |
|  | * Spalte 6, Zeile 4 - Spalte 15, Zeile 30 * | | H01S5/40 |
|  |  | | G01N21/17 |
|  | * Spalte 18, Zeile 59 - Spalte 19, Zeile 6 * | | G01N21/25 |
|  | ----- | | |
| X | US 2005/254539 A1 (KLIMEK DANIEL E [US]) 17. November 2005 (2005-11-17) | 1-14 | |
| Y | * Abbildungen 5-8 * | 15 | |
|  | * Absätze [0035] - [0057] * | | |
|  | ----- | | |
| X | US 2015/380894 A1 (YOSHINO MASAYA [JP] ET AL) 31. Dezember 2015 (2015-12-31) | 1-14 | |
| Y | * Abbildungen 1-3, 5-9 * | 15 | |
|  | ----- | | |
| X | US 2015/229106 A1 (YABE MITORU [JP]) 13. August 2015 (2015-08-13) | 1-14 | |
| Y | * Abbildungen 6-9 * | 15 | |
|  | * Absätze [0057] - [0074] * | | RECHERCHIERTE SACHGEBIETE (IPC) |
|  | ----- | | |
| X | US 2004/114648 A1 (NAGANO KAZUHIKO [JP] ET AL) 17. Juni 2004 (2004-06-17) | 1-14 | H01S |
| Y | * Abbildungen 1-9 * | 15 | G01N |
|  | ----- | | |
| Y | WO 2021/239262 A1 (DIAMONTECH AG [DE]) 2. Dezember 2021 (2021-12-02) | 15 | |
| A | * Seite 25, Zeile 28 - Seite 26, Zeile 7; Abbildung 3 * | 1-14 | |
|  | ----- | | |
|  | -/-- | | |

Der vorliegende Recherchenbericht wurde für alle Patentansprüche erstellt

| Recherchenort | Abschlußdatum der Recherche | Prüfer |
|---|---|---|
| München | 17. Dezember 2024 | Sauerer, Christof |

EPO FORM 1503 03.82 (P04C03)

Seite 1 von 2

Europäisches Patentamt
European Patent Office
Office européen des brevets

# EUROPÄISCHER RECHERCHENBERICHT

**Nummer der Anmeldung**

EP 24 19 2874

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | MIGUEL A. PLEITEZ ET AL: "In Vivo Noninvasive Monitoring of Glucose Concentration in Human Epidermis by Mid-Infrared Pulsed Photoacoustic Spectroscopy", ANALYTICAL CHEMISTRY, Bd. 85, Nr. 2, 26. Dezember 2012 (2012-12-26), Seiten 1013-1020, XP055499217, DOI: 10.1021/ac302841f | 15 | |
| A | * das ganze Dokument * ----- | 1-14 | |
| A | CN 110 429 475 A (SHANDONG HAIFU PHOTONICS TECH CO LTD) 8. November 2019 (2019-11-08) * das ganze Dokument * ----- | 1-15 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 17. Dezember 2024 | Sauerer, Christof |

EPO FORM 1503 03.82 (P04C03)

Seite 2 von 2

## ANHANG ZUM EUROPÄISCHEN RECHERCHENBERICHT
## ÜBER DIE EUROPÄISCHE PATENTANMELDUNG NR.

EP 24 19 2874

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-12-2024

| Im Recherchenbericht angeführtes Patentdokument | Datum der Veröffentlichung | Mitglied(er) der Patentfamilie | | Datum der Veröffentlichung |
|---|---|---|---|---|
| US 11881678 B1 | 23-01-2024 | KEINE | | |
| US 2005254539 A1 | 17-11-2005 | CA | 2567103 A1 | 08-12-2005 |
| | | EP | 1766742 A1 | 28-03-2007 |
| | | IL | 179326 A | 30-12-2010 |
| | | JP | 2007538404 A | 27-12-2007 |
| | | US | 2005254539 A1 | 17-11-2005 |
| | | WO | 2005117218 A1 | 08-12-2005 |
| US 2015380894 A1 | 31-12-2015 | CN | 104956555 A | 30-09-2015 |
| | | JP | 5920254 B2 | 18-05-2016 |
| | | JP | 2014175626 A | 22-09-2014 |
| | | US | 2015380894 A1 | 31-12-2015 |
| | | WO | 2014142147 A1 | 18-09-2014 |
| US 2015229106 A1 | 13-08-2015 | CA | 2863712 A1 | 13-08-2015 |
| | | CN | 104852273 A | 19-08-2015 |
| | | EP | 2928031 A1 | 07-10-2015 |
| | | JP | 6272067 B2 | 31-01-2018 |
| | | JP | 2015153840 A | 24-08-2015 |
| | | KR | 20150095544 A | 21-08-2015 |
| | | US | 2015229106 A1 | 13-08-2015 |
| US 2004114648 A1 | 17-06-2004 | JP | 2004179607 A | 24-06-2004 |
| | | US | 2004114648 A1 | 17-06-2004 |
| WO 2021239262 A1 | 02-12-2021 | KEINE | | |
| CN 110429475 A | 08-11-2019 | KEINE | | |

EPO FORM P0461

Für nähere Einzelheiten zu diesem Anhang : siehe Amtsblatt des Europäischen Patentamts, Nr.12/82

**EP 4 693 771 A1**

**IN DER BESCHREIBUNG AUFGEFÜHRTE DOKUMENTE**

**In der Beschreibung aufgeführte Patentdokumente**

- WO 2015193310 A1
- WO 2017097824 A1
- EP 2019064356 W
- WO 2019110597 A2
- WO 201709782 A1
- WO 201911059782 A